(19) Europäisches Patentamt
European Patent Office
Office européen des brevets

(11) EP 2 775 292 B1

(12) **EUROPEAN PATENT SPECIFICATION**

(45) Date of publication and mention
of the grant of the patent:
**03.08.2016 Bulletin 2016/31**

(51) Int Cl.:
**G01N 21/31** *(2006.01)*       **G01N 33/49** *(2006.01)*
**G01N 21/75** *(2006.01)*       **G01N 21/17** *(2006.01)*
**G01N 33/86** *(2006.01)*

(21) Application number: **14157809.6**

(22) Date of filing: **05.03.2014**

(54) **Blood coagulation analyzer and blood coagulation analyzing method**

Blutgerinnungsanalysegerät und Blutgerinnungsanalyseverfahren

Analyseur de coagulation de sang et procédé d'analyse de coagulation de sang

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.03.2013 JP 2013044778**

(43) Date of publication of application:
**10.09.2014 Bulletin 2014/37**

(73) Proprietor: **SYSMEX CORPORATION
Kobe-shi,
Hyogo 651-0073 (JP)**

(72) Inventors:
• **Yamaguchi, Keiichi
Hyogo, 651-0073 (JP)**

• **Takeuchi, Yasuhiro
Hyogo, 651-0073 (JP)**
• **Kurono, Hiroshi
Hyogo, 651-0073 (JP)**

(74) Representative: **Hoffmann Eitle
Patent- und Rechtsanwälte PartmbB
Arabellastraße 30
81925 München (DE)**

(56) References cited:
**EP-A1- 1 840 559      EP-A1- 2 259 069
EP-A2- 1 890 142      US-A1- 2007 222 973**

**Description**

FIELD OF THE INVENTION

The present invention relates to blood coagulation analyzers which analyze coagulation of blood by mixing a blood specimen and a reagent together, and relates to blood coagulation analyzing methods.

BACKGROUND

As a device to detect blood coagulation, US 2007/222973 discloses a device according to the preamble of independent device claim 1. As a method for detecting blood coagulation, there is a method in which coagulation measurement is performed by mixing sample plasma and a coagulation reagent together, and a scattered light detecting scheme and a transmitted light detecting scheme are known. For example, Japanese Laid-Open Patent Publication No. 2010-217059 discloses a blood coagulation analyzer using a scattered light detecting scheme. In this blood coagulation analyzer, a scattered light amount value is obtained by a measurement unit at a predetermined time interval, and a coagulation endpoint is detected based on temporal change in the scattered light amount value obtained after a predetermined reagent has been added to a blood sample. Then, a time point at which the scattered light amount value has reached 1/N (N is a predetermined value of 1 or greater) of the scattered light amount value at this coagulation endpoint is determined as a coagulation point, and an elapsed time from the time point of addition of the reagent to this coagulation point is calculated as a coagulation time.

Further, in the blood coagulation analyzer, whether the calculated coagulation time is normal is determined by a control section, and when it has been determined that the coagulation time is abnormal, measurement by the measurement unit is continued. Then, a time point every time after such continued measurement was started is assumed as a coagulation endpoint, and calculation of a coagulation time and determination of whether the coagulation time is appropriate is sequentially performed. When it has been determined that the coagulation time is normal, the measurement ends.

In the above-described blood coagulation analyzer, a determination line based on actual measurements is used in determination performed by the control section. That is, a distribution representing coagulation times and scattered light amounts at coagulation points obtained through normal coagulation reactions is obtained in advance through actual measurements, and based on this distribution, a determination line separating a distribution of normal coagulation reactions from a distribution of abnormal coagulation reactions is set. However, in this method, in order to increase the accuracy of the determination line, many samples are needed to be collected. If the number of samples is small, the accuracy of the determination line is reduced, causing a problem of reduced accuracy of determination of a coagulation time.

SUMMARY OF THE INVENTION

The scope of the present invention is defined by the appended claims.

(1) A first aspect of the present invention is a blood coagulation analyzer in accordance with claim 1.

According to this configuration, that blood coagulation reaction has not occurred can properly be determined based on the fact that optical property is different from wavelength to wavelength, and thus, an appropriate coagulation time can be calculated. Further, since the determination is performed based on optical information being a physical quantity different from wavelength to wavelength, complicated work such as collecting a lot of samples to be actually measured is not needed. Therefore, according to the blood coagulation analyzer of the present aspect, determination of appropriateness/inappropriateness of a blood coagulation time can be accurately performed without requiring complicated work. Thus, an appropriate coagulation time can be calculated.

According to this configuration, optical information can be more easily obtained.

As coagulation of blood advances, light from the measurement specimen changes. In this case, the amount of change of light differs from wavelength to wavelength. Therefore, by analyzing the difference (ratio or difference) between these amounts of changes, how far coagulation has advanced can be properly determined.

According to this configuration, a blood coagulation time can be more appropriately calculated.

According to this configuration, the amount of change of the light of the first wavelength and the amount of change of the light of the second wavelength can be more appropriately calculated.

As coagulation of blood advances, light from the measurement specimen changes. In this case, the amount of change of light differs from wavelength to wavelength. Therefore, by analyzing the ratio between these amounts of changes or the difference between these amounts of changes, how far coagulation has advanced can be properly determined.

(2) The blood coagulation analyzer according to (1), wherein
the obtaining section is configured to detect at least one of an intensity of light that has transmitted through the measurement specimen and an intensity of light that has been scattered by the measurement specimen.
According to this configuration, more appropriate optical information can be obtained. Therefore, determination of appropriateness/inappropriateness of a blood coagulation time can be more accurately performed, and a more appropriate coagulation time can be calculated.
(3) The blood coagulation analyzer according to (1, wherein
the controller is configured to determine appropriateness/inappropriateness of the calculated end time, by determining whether the relation between the amount of change of the light of the first wavelength and the amount of change of the light of the second wavelength satisfies a predetermined condition.
According to this configuration, an accurate end time can be obtained. Thus, a blood coagulation time can be more accurately calculated.
(4) The blood coagulation analyzer according to any one of claims (1) to (3), wherein
the controller is configured to calculate a coagulation time based on the optical information based on the light of the first wavelength and the optical information based on the light of the second wavelength.
According to this configuration, optical information for obtaining a blood coagulation time is commonly used as optical information for determining appropriateness/inappropriateness of the blood coagulation time. Thus, the number of types of light used in analysis can be reduced, and thus, the analysis process can be simplified.
(5) The blood coagulation analyzer according to any one of (1) to (4), wherein
the obtaining section is configured to at least obtain an absorbance based on the light of the first wavelength and an absorbance based on the light of the second wavelength, as the optical information based on the light of the first wavelength and the optical information based on the light of the second wavelength, and
the controller is configured to determine whether the relation between the amount of change of the light of the first wavelength and the amount of change of the light of the second wavelength satisfies a predetermined condition, based on the absorbance based on the light of the first wavelength and the absorbance based on the light of the second wavelength.
In this case, for example, the determination is performed based on the amount of change in the absorbance based on the light of the first wavelength and on the amount of change in the absorbance based on the light of the second wavelength. According to this configuration, the difference between the amount of change of the light of the first wavelength and the amount of change of the light of the second wavelength becomes evident. Thus, determination of appropriateness/inappropriateness of a blood coagulation time can be more accurately performed, and a more appropriate coagulation time can be calculated.
(6) The blood coagulation analyzer according to any one of (1) to (5), further comprising:

a notification section configured to make notification, when the controller has determined that the relation between the amount of change of the light of the first wavelength and the amount of change of the light of the second wavelength does not satisfy a predetermined condition, that a blood coagulation time cannot be appropriately obtained.

According to this configuration, the user can easily understand whether blood coagulation reaction occurred during the measurement, and can smoothly advance the procedure thereafter.
(7) The blood coagulation analyzer according to any one of (1) to (6), further comprising:

a reception section for receiving an instruction to perform re-measurement of a blood coagulation time, when the controller has determined that the relation between the amount of change of the light of the first wavelength and the amount of change of the light of the second wavelength does not satisfy a predetermined condition, wherein
upon the reception section receiving the instruction, the controller is configured to cause the obtaining section to perform re-measurement of a blood coagulation time, on a measurement specimen for re-measurement prepared by mixing a reagent to a blood specimen identical to the blood specimen for which it has been-determined that the relation between the amount of change of the light of the first wavelength and the amount of change of the light of the second wavelength does not satisfy a predetermined condition.

According to this configuration, in a case where blood coagulation reaction is abnormal in the current measurement, the user can cause the blood coagulation analyzer to perform re-measurement of a blood coagulation time smoothly and quickly.

(8) The blood coagulation analyzer according to (7), wherein

the obtaining section is configured to obtain the optical information, with a measurement time period for re-measurement set to be longer than an ordinary measurement time period.

According to this configuration, a measurement result of a correct blood coagulation time can be more easily obtained.

(9) A second aspect of the present invention is a blood coagulation analyzing method in accordance with claim 9.

According to this configuration, the determination is performed based on optical properties at different wavelengths. Therefore, determination of appropriateness/inappropriateness of a blood coagulation time can be accurately performed without requiring complicated work, and an appropriate coagulation time can be calculated.

BRIEF DESCRIPTION OF THE DRAWINGS

FIG. 1 is a perspective view showing an external structure of a blood coagulation analyzer according to a first embodiment;
FIG. 2 is a plan view of the inside of a measurement unit according to the first embodiment, viewed from above;
FIG. 3A shows a structure of a lamp unit according to the first embodiment;
FIG. 3B shows a structure of the vicinity of a filter part;
FIG. 3C shows a structure of an optical system of the lamp unit;
FIG. 4A shows a state where no cuvette is set in a holder of a detection part;
FIG. 4B shows a state where a cuvette is set in a holder of the detection part;
FIG. 5 shows a configuration of a measurement unit according to the first embodiment;
FIG. 6 shows a configuration of a control device according to the first embodiment;
FIG. 7A shows change in a transmitted light amount due to coagulation reaction according to the first embodiment;
FIG. 7B shows change in absorbance due to coagulation reaction according to the first embodiment;
FIG. 8A and FIG. 8B show flow charts representing a calculation process of a coagulation time according to the first embodiment;
FIG. 9 shows a screen on which an analysis result is displayed according to the first embodiment;
FIG. 10 shows a screen on which contents of an error are displayed according to the first embodiment;
FIG. 11 shows a screen on which an analysis result is displayed according to a second embodiment;
FIG. 12 is a flow chart showing a calculation process of a coagulation time according to the second embodiment;
FIG. 13A shows change in absorbance during measurement of the first time according to the second embodiment;
FIG. 13B shows change in absorbance during measurement of the second time according to the second embodiment;
FIG. 14 is a flow chart showing a calculation process of a coagulation time according to a third embodiment;
FIG. 15A and FIG. 15B are flow charts showing a calculation process of a coagulation time according to a fourth embodiment;
FIG. 16A and FIG. 16B are flow charts showing a calculation process of a coagulation time according to a modification;
FIG. 17A shows a structure of a detection part when scattered light is detected; and
FIG. 17B shows a structure of a detection part when scattered light and transmitted light are detected.

DETAILED DESCRIPTION OF THE PREFERRED EMBODIMENTS

The preferred embodiments of the present invention will be described hereinafter with reference to the drawings.
A blood coagulation analyzer according to the present embodiment performs analysis regarding coagulability of blood, by irradiating with light a measurement specimen prepared by adding a reagent to a sample (plasma), and by analyzing obtained transmitted light by use of a coagulation method, a synthetic substrate method, immunonephelometry, and an agglutination method. In the present embodiment, the present invention is applied to analysis (calculation of prothrombin time) that uses the coagulation method among the above analysis methods. Hereinafter, the blood coagulation analyzer according to the present embodiment will be described with reference to the drawings.

<First Embodiment>

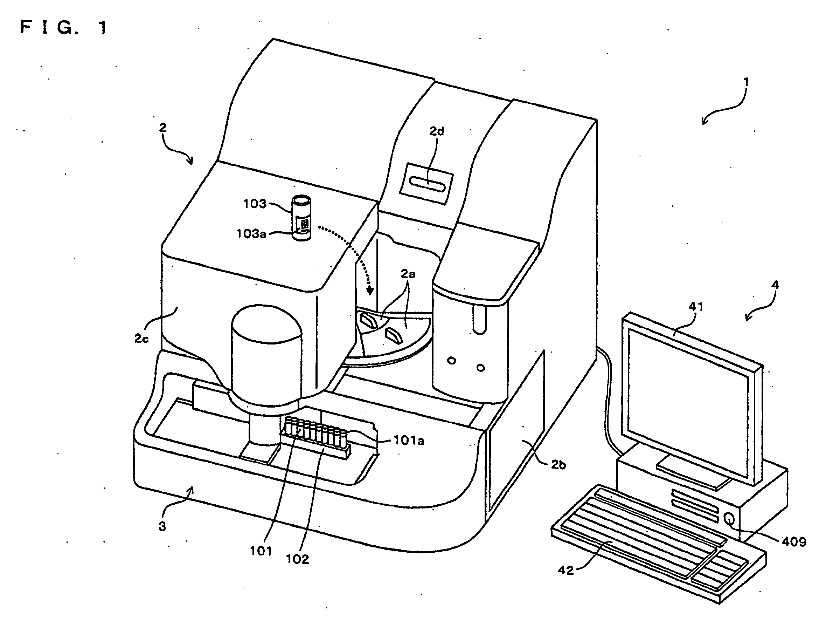

FIG. 1 is a perspective view showing an external structure of a blood coagulation analyzer 1.
The blood coagulation analyzer 1 includes a measurement unit 2 which optically measures components contained in a sample (plasma), a sample transporter 3 arranged to the front of the measurement unit 2, and a control device 4 which analyzes measurement data obtained by the measurement unit 2 and which provides instructions to the measurement unit 2.

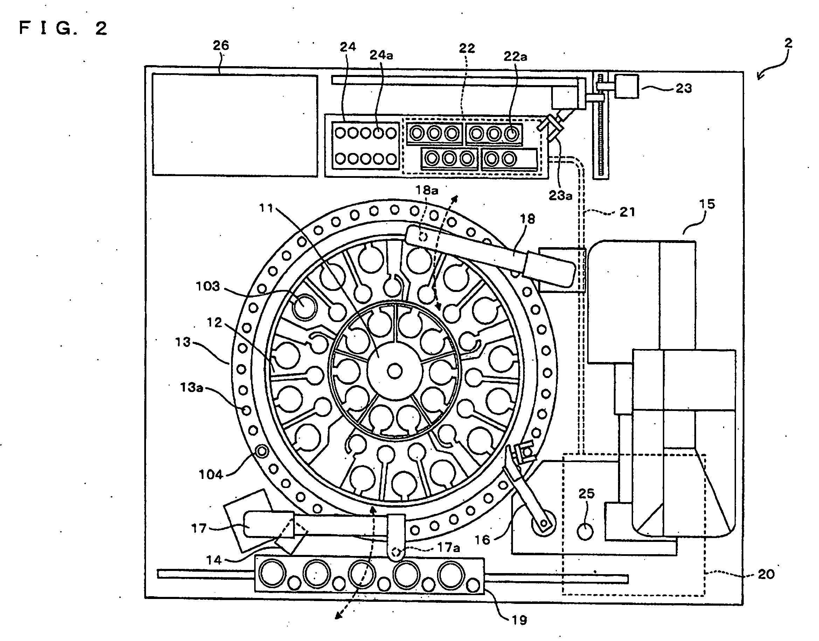

The measurement unit 2 is provided with lids 2a and 2b, a cover 2c, and a power button 2d. A user can open the lid 2a to replace reagent containers 103 set in reagent tables 11 and 12 (see FIG. 2) with new reagent containers 103, and to newly add other reagent containers 103 thereto. Each reagent container 103 has attached thereto a bar code label 103 a on which a bar code is printed, the bar code including the type of a reagent contained therein and a reagent ID composed of a serial number given to the reagent.

Further, the user can open the lid 2b to replace a lamp unit 20 (see FIG. 2) being a light source, and can open the cover 2c to replace a piercer 17a (see FIG. 2). The sample transporter 3 transports each of sample containers 101 held in a sample rack 102 to an aspiration position for the piercer 17a. Each sample container 101 is sealed with a lid 101a made of rubber.

Before using the blood coagulation analyzer 1, first, the user presses the power button 2d of the measurement unit 2 to activate the measurement unit 2, and presses a power button 409 of the control device 4 to activate the control device 4. Upon the control device 4 being activated, a log-on screen is displayed on a display unit 41 being a notification section. The user inputs a user name and a password on the log-on screen to log on the control device 4, and starts using the blood coagulation analyzer 1.

FIG. 2 is a plan view of the inside of the measurement unit 2, viewed from above.

As shown in FIG. 2, the measurement unit 2 includes the reagent tables 11 and 12, a cuvette table 13, a bar code reader 14, a cuvette supply part 15, a catcher 16, a sample dispensing arm 17, a reagent dispensing arm 18, an emergency sample setting part 19, the lamp unit 20, an optical fiber 21, a detection part 22, a cuvette transfer part 23, a heating part 24, a disposal hole 25, and a fluid part 26.

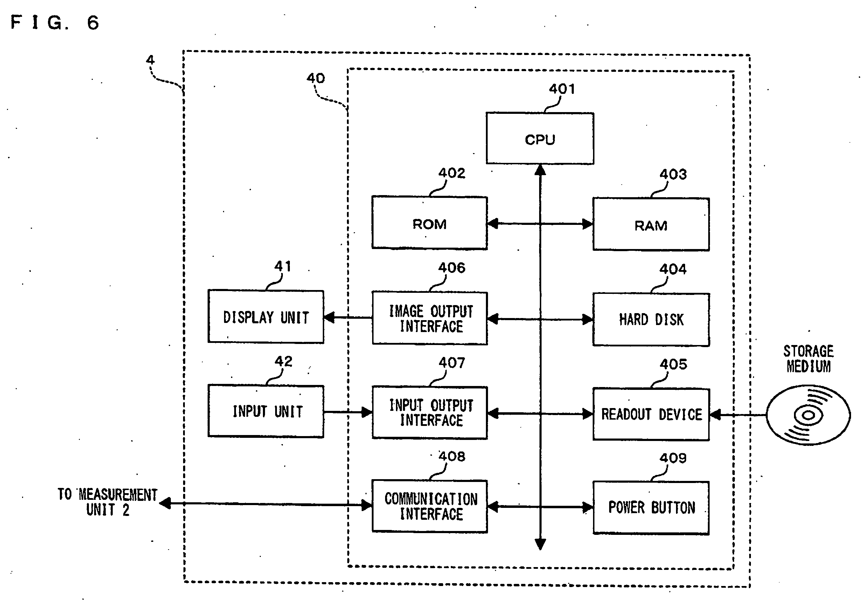

Each of the reagent tables 11 and 12 and the cuvette table 13 has an annular shape, and is configured to be able to rotate. On the reagent tables 11 and 12, reagent containers 103 are placed. The bar code of each reagent container 103 placed on the reagent tables 11 and 12 is read by the bar code reader 14. The information (the type of the reagent, the reagent ID) read from the bar code is inputted to the control device 4 and is stored in a hard disk 404 (see FIG. 6).

The cuvette table 13 has formed therein a plurality of holders 13a being a plurality of holes each capable of holding a cuvette 104 therein. New cuvettes 104 supplied into the cuvette supply part 15 by the user are sequentially transferred by the cuvette supply part 15, to be set in holders 13a in the cuvette table 13 by the catcher 16.

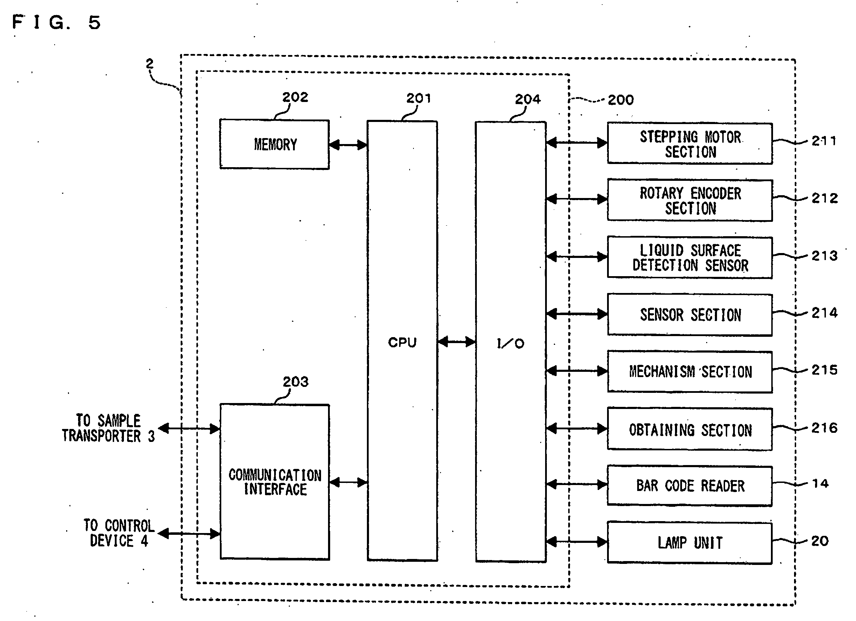

Stepping motors are connected to the sample dispensing arm 17 and the reagent dispensing arm 18, respectively, so as to allow the sample dispensing arm 17 and the reagent dispensing arm 18 to be able to move in the up-down direction and to rotate. To the tip of the sample dispensing arm 17, a piercer 17a is set whose tip is formed sharp such that the piercer 17a can puncture the lid 101a of a sample container 101. To the tip of the reagent dispensing arm 18, a pipette 18a is set. Unlike the piercer 17a, the tip of the pipette 18a is formed flat. Moreover, to the pipette 18a, a liquid surface detection sensor 213 (see FIG. 5) of a capacitance type is connected.

The lamp unit 20 supplies lights of five types of wavelengths to be used for detection of optical signals performed by the detection part 22. Light of the lamp unit 20 is supplied to the detection part 22 via the optical fiber 21.

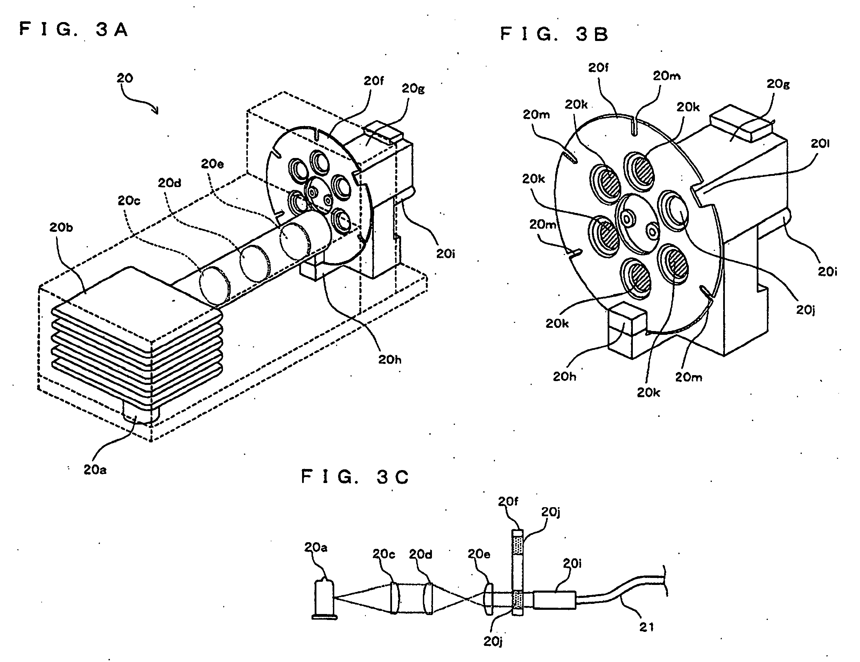

FIG. 3A shows a structure of the lamp unit 20, and FIG. 3B shows a structure of the vicinity of a filter part 20f, and FIG. 3C shows a structure of an optical system of the lamp unit 20.

As shown in FIG. 3A, the lamp unit 20 includes a halogen lamp 20a, a lamp case 20b, condensing lenses 20c to 20e, the filter part 20f having a disk shape, a motor 20g, a sensor 20h of a light transmissive-type, and an optical fiber coupler 20i.

The halogen lamp 20a is set to the lamp case 20b from below such that the filament thereof faces vertically upward. In the lamp case 20b, fins for releasing heat emitted from the halogen lamp 20a are formed. The condensing lenses 20c to 20e condense light emitted from the halogen lamp 20a. The condensing lenses 20c to 20e are arranged such that their optical axes are aligned with one another.

With reference to FIG. 3B, the filter part 20f has a disk shape and is supported at its center by the rotation shaft of the motor 20g. In the filter part 20f, six holes 20j are formed on the same circle at an interval of 60 degrees, and in five of the six holes 20j, optical filters 20k are mounted, respectively. Each optical filter 20k is a bandpass filter which transmits light in a predetermined wavelength band and cuts light in other wavelength bands. The center wavelengths of the transmitted wavelength bands of the five optical filters 20k are 340 nm, 405 nm, 575 nm, 660 nm, and 800 nm, respectively. It should be noted that the hole 20j with no optical filter 20k mounted therein is closed so as to prevent light from passing therethrough.

In the outer periphery of the filter part 20f, a cutout 201 and five slits 20m are provided. The cutout 201 is formed at a position corresponding to the hole 20j with no optical filter 20k mounted therein, and the slits 20m are formed at positions corresponding to the optical filters 20k, respectively. The cutout 201 is wider in the circumferential direction than each slit 20m. When the filter part 20f is rotated, the cutout 201 and the slits 20m pass a detection position of the sensor 20h.

The lamp unit 20 is configured such that, each time the filter part 20f is rotated by 60 degrees, the optical axis of the condensing lenses 20c to 20e runs through the center of a hole 20j of the filter part 20f. Therefore, as shown in FIG. 3C, light condensed by the condensing lenses 20c to 20e enters one of the five optical filters 20k each time the filter part 20f is rotated by 60 degrees. At the timing when the optical axis of the condensing lenses 20c to 20e runs through the center of each hole 20j of the filter part 20f, the slit 20m corresponding to the hole 20j faces the sensor 20h. Therefore, based on a detection signal of the sensor 20h, the timing when light enters each optical filter 20k can be detected.

Each of the condensing lenses 20d and 20e exhibits a function of a beam expander, and converts light from the lamp unit 20 into parallel light having a slightly smaller diameter than that of each hole 20j of the filter part 20f. Light that has transmitted through an optical filter 20k enters the optical fiber coupler 20i, and is distributed to a plurality of the optical fibers 21 connected to the optical fiber coupler 20i.

In the present embodiment, rotation of the filter part 20f is controlled so as to have a constant angular velocity. Accordingly, light in a different wavelength band enters the optical fiber coupler 20i at a constant time interval, and as a result, lights in different wavelength bands are supplied to the optical fibers 21 in a time division manner. For rotation control of the filter part 20f, a detection signal corresponding to the cutout 201 among detection signals obtained by the sensor 20h is used. That is, the motor 20g is controlled such that a detection signal corresponding to the cutout 201 is periodically detected. Further, for identification of light of which wavelength band is supplied to the optical fibers 21, a detection signal corresponding to each slit 20m is used. That is, since the five slits 20m are respectively formed at positions corresponding to the five optical filters 20k, the wavelength band of light supplied to the optical fibers 21 is identified by the order of a detection signal corresponding to a slit 20m from the detection signal corresponding to the cutout 201. During measurement, the filter part 20f is rotated at a velocity of about 10 rotations/second, for example.

With reference back to FIG. 2, light from the lamp unit 20 is supplied to the detection part 22 via the optical fibers 21. The detection part 22 is provided with a plurality of holders 22a each having a hole shape, and in each holder 22a, a cuvette 104 can be inserted. To each holder 22a, an end portion of an optical fiber 21 is mounted such that a cuvette 104 held in the holder 22a can be irradiated with light from the optical fiber 21. The detection part 22 irradiates each cuvette 104 with light supplied from the lamp unit 20, via a corresponding optical fiber 21, to detect an amount of light transmitting through the cuvette 104.

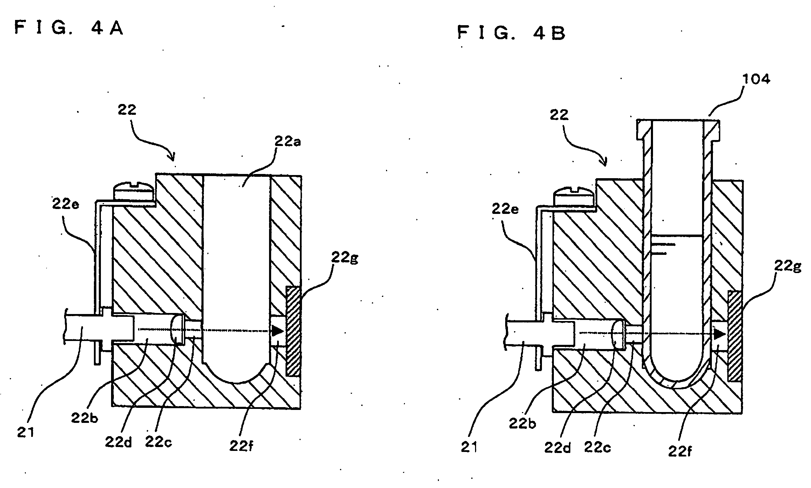

Each of FIG. 4A and FIG. 4B is a cross-sectional view showing a structure of the vicinity of a holder 22a. FIG. 4A shows a state where no cuvette 104 is set in the holder 22a, and FIG. 4B shows a state where a cuvette 104 is set in the holder 22a. Each of FIG. 4A and FIG. 4B shows a cross-sectional view obtained when the detection part 22 is vertically cut with a plane passing through the center of the holder 22a.

It should be noted that each of FIG. 4A and FIG. 4B shows the structure of one of the plurality of holders 22a arranged in the detection part 22, but the other holders 22a also have the same structure.

With reference to FIG. 4A, in the detection part 22, a hole 22b, which is round and into which a tip of the optical fiber 21 is inserted, is formed, and further, a communication hole 22c, which is round and which allows the hole 22b to communicate with the holder 22a, is formed. The diameter of the hole 22b is greater than the diameter of the communication hole 22c. In an end of the hole 22b, a lens 22d which condenses light from the optical fiber 21 is arranged. Further, in an inner wall surface of the holder 22a, a hole 22f is formed at a position opposed to the communication hole 22c, and at the back of the hole 22f, a light detector 22g as a light receiving part is arranged. The light detector 22g outputs an electric signal corresponding to an amount of received light. Light that has transmitted through the lens 22d is condensed at a light receiving face of the light detector 22g, via the communication hole 22c, the holder 22a, and the hole 22f. The optical fiber 21 is stopped by means of a plate spring 22e so as not to slip off, with an end portion of the optical fiber 21 being inserted in the hole 22b.

With reference to FIG. 4B, when a cuvette 104 is held in the holder 22a, light condensed by the lens 22d transmits through the cuvette 104 and the specimen contained in the cuvette 104, to enter the light detector 22g. When blood coagulation reaction advances in the specimen, turbidity of the specimen increases. Associated with this, the amount of light (transmitted light amount) that transmits through the specimen decreases, and the level of a detection signal of the light detector 22g decreases.

As described above, from the optical fiber 21, the five types of light in different wavelength bands are emitted in a time division manner. Lights of the respective wavelengths are used in measurement by different analysis methods, respectively.

In the coagulation method, light of 660 nm wavelength is used, and transmitted light from the specimen is detected by the light detector 22g, whereby a time period in which fibrinogen is converted into fibrin is analyzed. Measurement items for the coagulation method include PT (prothrombin time), APTT (activated partial thromboplastin time), Fbg (amount of fibrinogen), and the like. In the synthetic substrate method, light of 405 nm wavelength is used, and transmitted light from the specimen is detected by the light detector 22g. Measurement items for the synthetic substrate method include ATIII, $\alpha$2-PI ($\alpha$2-plasmin inhibitor), PLG (plasminogen), and the like. In the immunonephelometry, light of 800 nm wavelength is used, and transmitted light from the specimen is detected by the light detector 22g. Measurement items for the immunonephelometry include D-dimer, FDP, and the like. In the agglutination method, light of 575 nm wavelength is used, and transmitted light from the specimen is detected by the light detector 22g.

In the present embodiment, in analyses by the coagulation method, the immunonephelometry, and the agglutination method, light amounts of transmitted light from the specimen are used. However, in these analysis methods, analyses can be performed by using light amounts of scattered light instead of light amounts of transmitted light.

In each of the analysis methods, among signals outputted from the light detector 22g, a signal based on light of a corresponding wavelength is extracted to be used in analysis. That is, as described above, from the optical fiber 21, lights of different wavelengths are emitted in a time division manner, and thus, also from the light detector 22g, signals corresponding to lights of the respective wavelengths are outputted in a time division manner. In an analysis process based on each analysis method, among signals outputted in a time division manner as described above, a signal corresponding to a wavelength used in its analysis is extracted and processing is performed.

With reference back to FIG. 2, upon a sample container 101 being transported to a predetermined position by the sample transporter 3 (see FIG. 1), the piercer 17a is located immediately above the sample container 101 through rotation of the sample dispensing arm 17. Then, the sample dispensing arm 17 is moved downwardly, the piercer 17a pierces the lid 101 a of the sample container 101, and the sample contained in the sample container 101 is aspirated by the piercer 17a. In a case where a sample that needs to be immediately analyzed is set in the emergency sample setting part 19, the piercer 17a aspirates the sample that needs to be immediately analyzed, ahead of samples supplied from the sample transporter 3. The sample aspirated by the piercer 17a is discharged into an empty cuvette 104 on the cuvette table 13.

The cuvette 104 into which the sample has been discharged is transferred by a catcher 23a of the cuvette transfer part 23, from the holder 13a in the cuvette table 13 to a holder 24a in the heating part 24. The heating part 24 heats the sample contained in the cuvette 104 set in the holder 24a to a predetermined temperature (for example, about 37°C). Upon completion-of-heating-of-the sample by the heating part 24, the cuvette 104 is gripped by the catcher 23a again. Then, the cuvette 104 is located at a predetermined position while being gripped by the catcher 23a, and in this state, a reagent aspirated by the pipette 18a is discharged into the cuvette 104.

For dispensing of a reagent performed by the pipette 18a, first, the reagent table 11, 12 is rotated, and a reagent container 103 containing a reagent corresponding to a measurement item is transported to an aspiration position for the pipette 18a. Then, based on a sensor for detecting an origin position, the position in the up-down direction of the pipette 18a is brought to the origin position, and then, the pipette 18a is lowered until the liquid surface detection sensor 213 detects that the lower end of the pipette 18a has come into contact with.the liquid surface of the reagent. When the lower end of the pipette 18a has been brought into contact with the liquid surface of the reagent, the pipette 18a is further lowered to an extent that allows aspiration of a necessary amount of the reagent. Then, lowering of the pipette 18a is stopped, and the reagent is aspirated by the pipette 18a. The reagent aspirated by the pipette 18a is discharged into the cuvette 104 gripped by the catcher 23a. Then, due to a vibration function of the catcher 23a, the sample and the reagent in the cuvette 104 are stirred. Accordingly, a measurement specimen is prepared.

Then, the cuvette 104 containing the measurement specimen is transferred to a holder 22a in the detection part 22 by the catcher 23a. As described above, the detection part 22 irradiates the cuvette 104 with light supplied from the lamp unit 20 and obtains transmitted light amounts as optical information. The obtained optical information is transmitted to the control device 4. The control device 4 performs analysis based on the optical information and causes the display unit 41 to display an analysis result.

The cuvette 104 for which measurement has ended and is no longer needed is transported by the cuvette table 13 and discarded by the catcher 16 into the disposal hole 25. It should be noted that, during measurement operation, the piercer 17a and the pipette 18a are cleaned as appropriate with a liquid such as a cleaning solution supplied from the fluid part 26.

FIG. 5 shows a configuration of the measurement unit 2.

The measurement unit 2 includes a control section 200, a stepping motor section 211, a rotary encoder section 212, the liquid surface detection sensor 213, a sensor section 214, a mechanism section 215, an obtaining section 216, the bar code reader 14, and the lamp unit 20. The control section 200 includes a CPU 201, a memory 202, a communication interface 203, and an I/O interface 204.

The CPU 201 executes computer programs stored in the memory 202. The memory 202 is implemented by a ROM, a RAM, a hard disk, and the like. The CPU 201 drives the sample transporter 3 and transmits/receives instruction signals and data to/from the control device 4, via the communication interface 203. Further, the CPU 201 controls components in the measurement unit 2 and receives signals outputted from the components, via the I/O interface 204.

The stepping motor section 211 includes stepping motors for respectively driving the reagent tables 11 and 12, the cuvette table 13, the catcher 16, the sample dispensing arm 17, the reagent dispensing arm 18, and the cuvette transfer part 23. The rotary encoder section 212 includes rotary encoders which output pulse signals corresponding to amounts of rotation displacements of the respective stepping motors included in the stepping motor section 211.

The liquid surface detection sensor 213 is connected to the pipette 18a set at the tip of the reagent dispensing arm 18, and detects that the lower end of the pipette 18a has come into contact with the liquid surface of a reagent. The sensor section 214 includes a sensor which detects that the position in the up-down direction of the pipette 18a has been brought to the origin position, and a sensor which detects that the power button 2d has been pressed. The mechanism section 215 includes mechanisms for driving the cuvette supply part 15, the emergency sample setting part 19, the heating part 24, and the fluid part 26, and a pneumatic source which supplies pressure to the piercer 17a and the pipette 18a so as to allow dispensing operation of the piercer 17a and the pipette 18a. The obtaining section 216 includes the detection part 22.

FIG. 6 shows a configuration of the control device 4.

The control device 4 is implemented by a personal computer, and includes a body 40, the display unit 41, and an input unit 42. The body 40 includes a CPU 401, a ROM 402, a RAM 403, the hard disk 404, a readout device 405, an image output interface 406, an input output interface 407, a communication interface 408, and the power button 409.

The CPU 401 executes computer programs stored in the ROM 402 and computer programs loaded onto the RAM 403. The RAM 403 is used for reading out computer programs stored in the ROM 402 and the hard disk 404. The RAM 403 is also used as a work area for the CPU 401 when the CPU 401 executes these computer programs.

The hard disk 404 has stored therein an operating system, computer programs to be executed by the CPU 401, and contents of settings of the control device 4. The readout device 405 is implemented by a CD drive, a DVD drive, or the like, and can read out computer programs and data stored in a storage medium such as a CD, a DVD, or the like.

The image output interface 406 outputs an image signal corresponding to image data to the display unit 41, and the display unit 41 displays an image based on the image signal outputted from the image output interface 406. The user inputs an instruction via the input unit 42, and the input output interface 407 receives a signal inputted via the input unit 42. The communication interface 408 is connected to the measurement unit 2. The CPU 401 transmits/receives instruction signals and data to/from the measurement unit 2, via the communication interface 408.

With reference to FIG. 5, during measurement operation, the CPU 201 of the measurement unit 2 temporarily stores, in the memory 202, data obtained by digitizing a detection signal outputted from each light detector 22g (see FIG. 4B), as optical information. The storage region of the memory 202 is divided into areas for the respective holders 22a. In each area, data obtained when a cuvette 104 held in a corresponding holder 22a is irradiated with light of a predetermined wavelength is sequentially stored as optical information. That is, from the light detector 22g of each holder 22a, as described above, detection signals corresponding to lights of the five types of wavelengths are outputted in a time division manner. The CPU 201 extracts, from among the five types of detection signals, a detection signal of a wavelength to be used in analysis of the measurement specimen in the cuvette 104 held in a holder 22a, and sequentially stores data obtained by digitizing the extracted detection signal, into an area corresponding to the holder 22a on the memory 202. In more detail, a detection signal at a timing when each slit 20m shown in FIG. 3B passes the sensor 20h is digitized to be stored in the memory 202. Accordingly, in a case where the filter part 20f is rotated at a velocity about 10 rotations/second, data corresponding to each wavelength is obtained ten times per second as optical information, to be stored in the memory 202.

In the present embodiment, in obtaining a prothrombin time through analysis based on the coagulation method, data obtained at the time of irradiation with light of 660 nm wavelength, and in addition, data obtained at the time of irradiation with light of 575 nm wavelength are used as optical information, as described later. Therefore, in a case where analysis to be performed on the measurement specimen contained in a cuvette 104 is to obtain a prothrombin time through analysis based on the coagulation method, both of data obtained at the time of irradiation with light of 660 nm wavelength and data obtained at the time of irradiation with light of 575 nm wavelength are stored as optical information in the corresponding area on the memory 202.

In this manner, data is sequentially stored in the memory 202 for a predetermined measurement time period. When the measurement time period has elapsed, the CPU 201 stops storing data into the memory 202, and transmits the stored data to the control device 4 via the communication interface 203. The control device 4 processes the received data to perform analysis for a predetermined item, and causes the display unit 41 to display an analysis result.

Hereinafter, obtainment of a prothrombin time through an analysis process based on the coagulation method according to the present embodiment will be described.

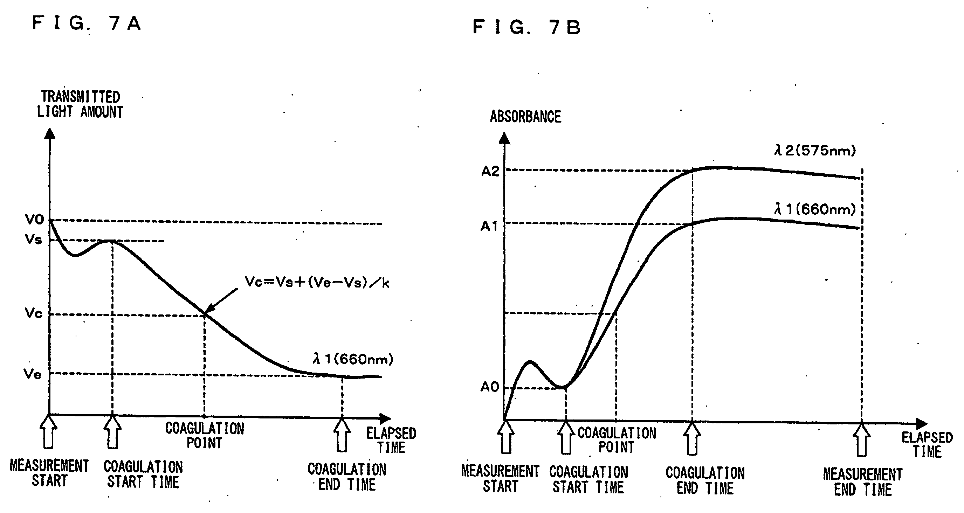

FIG. 7A schematically shows an example of a reaction curve based on the coagulation method. In FIG. 7A, the horizontal axis represents elapsed time, and the vertical axis represents transmitted light amount. The transmitted light amount on the vertical axis is the transmitted light amount received by a light detector 22g, and is expressed by a digital value of a detection signal outputted from the light detector 22g. The elapsed time on the horizontal axis is an elapsed time from the time point when a reagent was added (reagent addition time). FIG. 7A illustrates change in the transmitted light amount when a measurement specimen is irradiated with light of 660 nm wavelength which is used when a prothrombin time is to be obtained.

In the present embodiment, as described above, a reagent is added to the sample (plasma) to prepare a measurement specimen, and then, the cuvette 104 is transferred to a holder 22a and measurement is started. Accordingly, a time lag occurs between the reagent addition time and the measurement start time. However, during this time lag, normally, blood coagulation reaction does not occur. Therefore, occurrence of the time lag does not affect measurement of a coagulation point (obtainment of a prothrombin time). However, the elapsed time is measured from the reagent addition time, not from the measurement start time. For convenience, in FIG. 7A, the reagent addition time is not shown on the time axis (horizontal axis), and the reaction curve on and after the measurement start time is shown.

When blood coagulation reaction has started after the addition of the reagent, turbidity of the measurement specimen increases due to coagulation of blood, and the transmitted light amount gradually decreases. In the reaction curve shown in FIG. 7A, the timing when the reaction curve begins to decline is the start time of the blood coagulation reaction. Thereafter, until the blood coagulation reaction is saturated, the transmitted light amount gradually attenuates. Saturation of the blood coagulation reaction is detected by the amount of change in the transmitted light amount per unit time period having become a predetermined threshold value or lower. In FIG. 7A, the timing when the blood coagulation reaction becomes saturated is shown as a coagulation end time.

In general, a coagulation point of blood is set at a position corresponding to 1/k of the amplitude of the reaction curve from before the start of the coagulation reaction until the end of the coagulation reaction shown in FIG. 7A. In the example in FIG. 7A, the elapsed time at the time when the transmitted light amount (a digital value of a detection signal) has become Vc indicates the coagulation point. Here, if the transmitted light amount at a coagulation start time is defined as Vs and the transmitted light amount at the coagulation end time is defined as Ve, Vc is expressed by the following formula.

$$Vc = Vs + (Ve - Vs)/k \quad …(1)$$

For example, when k is set to be 2, the coagulation point is set at a position corresponding to 1/2 of the amplitude of the reaction curve from before the start of the coagulation reaction until the end of the coagulation reaction. The elapsed time from addition of the reagent to the coagulation point is a prothrombin time (PT).

In the present embodiment, further, appropriateness/inappropriateness of the coagulation end time is determined based on absorbance. Here, an absorbance At at an elapsed time t is determined by the following formula.

$$At = -\log_{10}(Vt/V0) …(2)$$

V0 is the transmitted light amount (a digital value of a detection signal from the light detector 22g) at an initial stage of the measurement. Here, the transmitted light amount at the measurement start time is set as V0. Further, Vt is the transmitted light amount at the elapsed time t (a digital value of a detection signal).

FIG. 7B schematically shows a waveform of absorbance which changes as time elapses.

As shown in formula (2) above, an absorbance is determined through logarithmic conversion of a transmitted light amount. Thus, the waveform in FIG. 7B showing change in absorbance is steep compared with the waveform in FIG. 7A. In FIG. 7B, along with a waveform of absorbance based on light of 660 nm wavelength used in calculation of a prothrombin time (PT), a waveform of absorbance based on light of 575 nm wavelength is shown. Moreover, in FIG. 7B, the measurement start time, the coagulation start time, the coagulation point, and the coagulation end time shown in FIG. 7A are shown, and further, a measurement end time is shown. A0 is the absorbance based on light of 660 nm wavelength at the coagulation start time. A1 is the absorbance based on light of 660 nm wavelength at the coagulation end time. A2 is the absorbance based on light of 575 nm wavelength at the coagulation end time.

An absorbance is a parameter quantity showing how much light attenuates while light passes through a measurement specimen. In general, factors which cause light to attenuate include scattering, reflecting, and absorbing of light by substances in the measurement specimen. Therefore, an absorbance is determined by a scattered light intensity, a reflected light intensity, and an absorbed light intensity of light in the measurement specimen.

Here, when a scattered light intensity is examined, light scattering includes Rayleigh scattering and Mie scattering The particle size of fibrinogen is 9 nm and is smaller than the wavelength (here, 660 nm, 575 nm) of light used in the measurement. Thus, it is considered that scattering of light at the measurement specimen is mainly Rayleigh scattering. In general, a scattered light intensity caused by Rayleigh scattering is represented by a scattering coefficient $k_3$ shown in the following formula. In the following fonnula" n is the number of .particles, d is a particle size, m is a reflection coefficient, and z, is a wavelength.

[Math.1]

$$k_3 = \frac{2\pi^5}{3} n \left( \frac{m^2 - 1}{m^2 + 2} \right)^2 \frac{d^6}{\lambda^4} \qquad \cdots \quad (3)$$

As seen in formula (3), a scattered light intensity due to Rayleigh scattering is directly proportional to the sixth power of the particle size d. Therefore, when coagulation of blood advances in the measurement specimen and the particle size increases (when the conversion rate from fibrinogen to fibrin increases), scattered light rapidly increases, and in association with this, the transmitted light amount decreases and the absorbance increases. Moreover, as seen from formula (3), a scattered light intensity due to Rayleigh scattering is inversely proportional to the fourth power of the wavelength X. Therefore, the shorter the wavelength of light emitted to the measurement specimen is, the higher the scattered light intensity becomes, the less the transmitted light amount becomes, and the higher- the absorbance becomes.

In a case where blood coagulation reaction has occurred in a measurement specimen, due to a factor that the scattered light intensity is different from wavelength to wavelength as described above, the absorbances A 1 and A2 at the coagulation end time differ from each other to a relatively great extent as shown in FIG. 7B. As described above, factors that cause the absorbances AI. and A2 to differ from each other include reflected light intensities and absorbed light intensities in addition to scattered light intensities. Thus, the difference between the absorbances A1 and A2 has a magnitude resulting from all of these factors combined together.

The present inventors have found through verification that, among combinations of two wavelengths (340 nm, 405 nm, 575 nm, 660 nm, and 800 nm) of light supplied from the lamp unit 20, with respect to a combination of 660 nm wavelength and 575 nm wavelength, the difference between the absorbances A1 and A2 on reaction curves due to coagulation reaction is evident compared with the difference between the absorbances A1 and A2 on reaction curves due to the other reactions. Therefore, when whether blood coagulation reaction has occurred is determined based on a difference between absorbances, it can be said that it is most appropriate to use absorbances based on light of 660 nm wavelength and light of 575 nm wavelength as shown in FIG. 7B.

In a case where blood coagulation reaction has not occurred, the difference between the absorbances A and A2 is reduced compared with the case where blood coagulation reaction has occurred. Therefore, by comparing a value (for example, the difference between A1 and A2, the ratio between A1 and A2, or the like) representing the difference between the absorbances A1 and A2 with a predetermined threshold value, whether blood coagulation reaction has occurred can be appropriately determined. Further, whether the coagulation end time is a true one, and whether a true coagulation point can be calculated based on the coagulation end time can be appropriately determined.

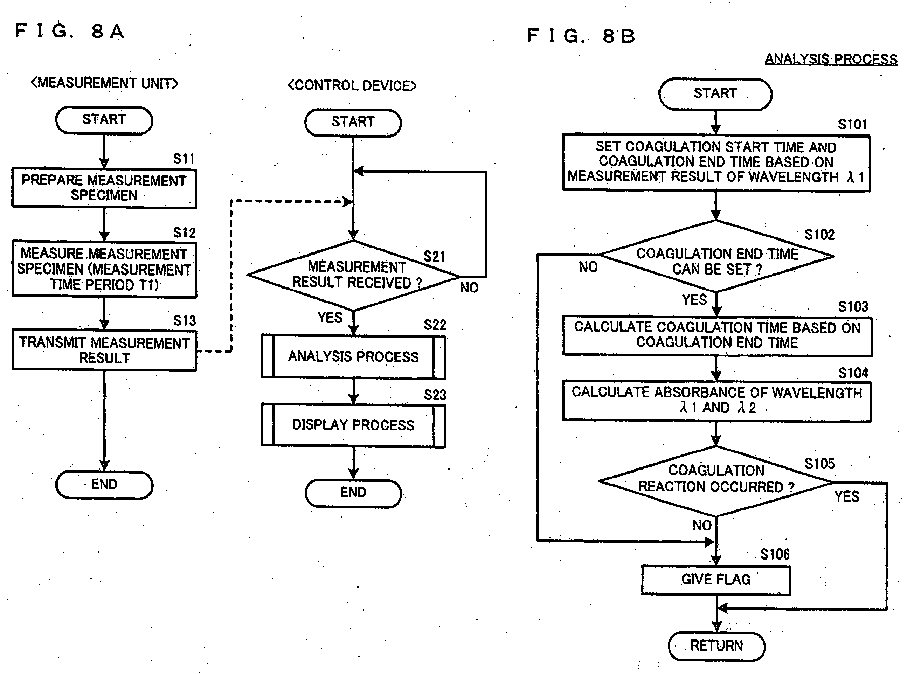

FIG. 8A and FIG. 8B show flow charts representing a measurement process of a prothrombin time (PT) according to the present embodiment. In the flow charts in FIG. 8A, the process in the measurement unit 2 is mainly performed under control of the CPU 201 of the measurement unit 2, and the process in the control device 4 is mainly performed under control of the CPU 401 of the control device 4.

With reference to FIG. 8A, upon start of the measurement process, the measurement unit 2 aspirates a sample (plasma) from a sample container 101 and dispenses the aspirated sample into an empty cuvette 104 on the cuvette table 13, as described above. Next, the measurement unit 2 transfers, to the heating part 24, the cuvette 104 into which the sample has been dispensed, heats the sample in the cuvette 104 to a predetermined temperature (for example, 37°C), and then adds a reagent to the cuvette 104 to prepare a measurement specimen (S11). The measurement unit 2 starts measuring a time period from the time point when the reagent was added to the cuvette 104.

Then, the measurement unit 2 transfers, to the detection part 22, the cuvette 104 into which the reagent has been added, irradiates the cuvette 104 with light, and measures the measurement specimen (SI2). As described above, in this measurement, the transmitted light amount being data based on light of 660 nm wavelength and the transmitted light amount being data based on light of 575 nm wavelength are sequentially stored into the memory 202 during a measurement time period T1. At this time, data of each wavelength is stored in the memory 202, in association with an elapsed time from the reagent addition time. Thereafter, when the measurement time period T1 has elapsed, the measurement unit 2 stops measurement on the measurement specimen, and transmits, to the control device 4, data being measurement results based on the above two wavelengths and being stored in the memory 202 (S13).

Accordingly, when the control device 4 has received the data being the measurement results from the measurement unit 2 (S21: YES), the control device 4 performs an analysis process on the received measurement results, to calculate a prothrombin time (PT) of the measurement specimen (S22).

FIG. 8B is a flow chart showing contents of the analysis process performed in S22.

The control device 4 sets a coagulation start time and a coagulation end time based on the transmitted light amount being data based on light of 660 nm wavelength, among the received measurement results (S101). As shown in FIG. 7A, the coagulation start time is set at a time point from which the transmitted light amount begins to decline.

The coagulation end time is set at a time point at which the slope of the transmitted light amount after the coagulation start time becomes substantially flat. Whether the slope of the transmitted light amount has become substantially flat is determined based on whether the slope of the transmitted light amount has reached a predetermined threshold value. In a case where a plurality of pairs of a coagulation start time and a coagulation end time are obtained in the measurement time period T1, a pair having a greatest amount of change in the transmitted light amount from a coagulation start time to a coagulation end time is selected for calculation of a coagulation point.

Next, the control device 4 determines whether the coagulation end time has been able to be set based on the transmitted light amount being data based on light of 660 nm wavelength S102). Specifically, when the slope of the transmitted light amount has not reached the predetermined threshold value (has not become substantially flat) after the coagulation start time, it is determined that the coagulation end time has failed to be set. When the coagulation end time has failed to be set (S102: NO), the control device 4 causes the memory 202 to hold a flag indicating that blood coagulation reaction has not occurred (S106). When the coagulation end time has been able to be set (S102: YES), the control device 4 sets a coagulation point based on the coagulation end time, and calculates a blood coagulation time (prothrombin time) (S103). The coagulation point is set at a position corresponding to 1/k of the amplitude of the waveform from before the start of the coagulation reaction until the end of the coagulation reaction, as described with reference to FIG. 7A.

Thereafter, with respect to the transmitted light amount being data based on light of 660 nm wavelength, the control device 4 calculates an absorbance A10 at the coagulation start time and an absorbance A11 at the coagulation end time, and further, with respect to the transmitted light amount being data based on light of 575 nm wavelength, the control device 4 calculates an absorbance A20 at the coagulation start time and an absorbance A21 at the coagulation end time (S104). Then, the control device 4 determines whether the coagulation end time set in S101 is based on blood coagulation reaction, based on the following determination condition (S105).

$$(A21 - A20) / (A11 - A10) \geq Ash \quad \ldots(4)$$

That is, an amount of change in absorbance from the coagulation start time to the coagulation end time is determined for each of these two wavelengths. Then, when the ratio of the determined amounts of changes is greater than or equal to a threshold value Ash, it is determined that the coagulation end time set in S101 is based on coagulation reaction. As described above, when blood coagulation reaction has occurred, the difference between absorbances of the respective wavelengths at the coagulation end time becomes large, and when blood coagulation reaction has not occurred, the difference between absorbances of the respective wavelengths at the coagulation end time is reduced, compared with the case where blood coagulation reaction has occurred. Therefore, by comparing the ratio of the amounts of changes in absorbance of the respective wavelengths with a threshold value, it is possible to appropriately determine whether the coagulation end time is based on blood coagulation reaction.

Upon determining that the coagulation end time set in S101 is not based on blood coagulation reaction (S105: NO), the control device 4 causes the memory 202 to hold the flag indicating that blood coagulation reaction has not occurred (S106). On the other hand, upon determining that the coagulation end time set in S101 is based on blood coagulation reaction (S105: YES), the control device 4 ends the analysis process without giving the flag.

With reference back to FIG. 8A, after performing the analysis process (S22), the control device 4 performs a display process of an analysis result (S23). In this display process, depending on whether the flag is given in S106 in FIG. 8B, contents of a screen D1 displayed on the display unit 41 are adjusted.

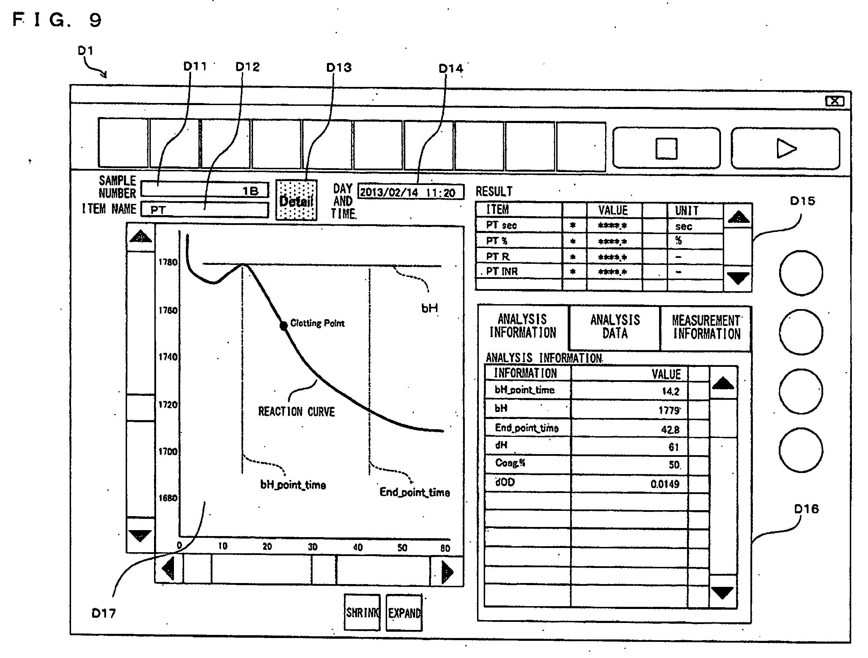

FIG. 9 shows the screen D1 when the flag has been given.

The screen D1 includes a region D11 in which a sample number is displayed, a region D12 in which a measurement item name is displayed, a button D13 for displaying a detailed screen, a region D 14 in which measurement day and time are displayed, a region in which a measurement result is displayed, a region D16 in which analysis information is displayed, and a region D17 in which a reaction curve is displayed.

In the region D15, measurement items and measurement values are displayed. In the region D15, "PT sec" is a prothrombin time. In the region D15, in addition to the prothrombin time (PT sec), values obtained by converting the prothrombin time into predetermined parameter values (PT%, PT R, PT INR) are displayed.

In the region D16, analysis items and their values are displayed. In the region D16, "bH point time" is the coagulation start time, "bH" is the transmitted light amount at the coagulation start time, "End point time" is the coagulation end time, "dH" is the difference between the transmitted light amount at the coagulation start time and the transmitted light amount at the coagulation end time, "Coag%" is a set value for setting a coagulation point, "dOD" is an average slope of the waveform in a period from the coagulation start time to the coagulation end time obtained when the reaction curve of the transmitted light amount has been converted into a reaction curve of absorbance. The value set as "Coag%" indicates at what percentage position of the level of the transmitted light amount at the coagulation start time relative to the transmitted light amount at the coagulation end time the coagulation point is to be set. In the region D17, a reaction curve is shown. The horizontal axis represents time period (second), and the vertical axis represents transmitted light amount (digital value). In the region D17, "Clotting Point" is the coagulation point.

In a case where the flag has been given in S106 in FIG. 8B, the items of the measurement values in the region D15 are masked as shown in FIG. 9, and the measurement values are not displayed. In a case where the flag has not been given, in the respective items in the region D15, measurement values are displayed. Moreover, in a case where the items of the measurement values in the region D15 are masked, the button D13 is displayed in a predetermined color and enabled. By operating the button D13, the user can confirm contents of an error. In a case where the items of the measurement values in the region D15 are not masked, the button D13 is disabled.

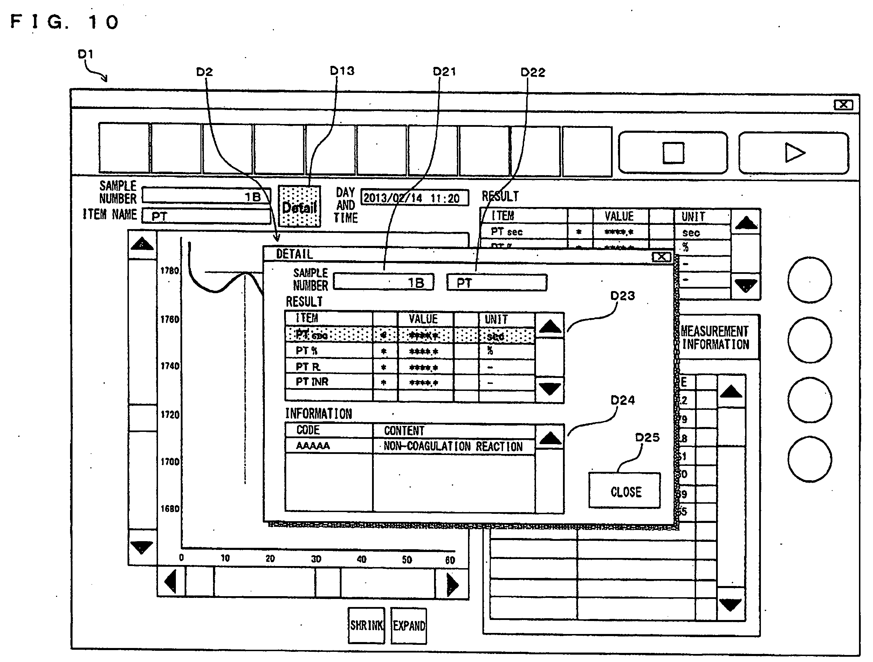

FIG. 10 shows a state of a screen when the button D13 has been operated.

As shown in FIG. 10, when the button D13 has been operated, a screen D2 is displayed so as to overlap the screen D1. The screen D2 includes a region D21 in which a sample number is displayed, a region D22 in which a measurement item name is displayed, a region D23 in which a measurement result is displayed, a region D24 in which error information is displayed, and a button D25 for closing the screen D2. In a case where the flag has been given in S106 in FIG. 8B, "non-coagulation reaction" is displayed in the region D24. Accordingly, the user can know that blood coagulation reaction did not occur within the measurement time period T1.

As described above, according to the present embodiment, based on the fact that absorbance indicating an optical property is different from wavelength to wavelength, appropriateness/inappropriateness of a blood coagulation time (PT) is determined. Thus, that blood coagulation reaction has not occurred can be properly determined, and a highly accurate, determination result can be obtained. Moreover, since the determination is performed based on optical information being a physical quantity different from wavelength to wavelength, complicated work such as collecting a lot of samples to be actually measured is not needed. Therefore, according to the present embodiment, determination of appropriateness/inappropriateness of a blood coagulation time (PT) can be accurately performed without requiring complicated work.

According to the present embodiment, as shown in formula (4) above, whether blood coagulation reaction has occurred is determined based on the ratio of the amounts of changes in absorbance, and thus, even when the amount of a measurement specimen is little, a highly accurate determination result can be obtained.

According to the present embodiment, light of 660 nm wavelength for obtaining a blood coagulation time (PT) is commonly used as light for determining whether blood coagulation reaction has occurred. Thus, the number of types of light used in analysis can be reduced, and thus, the analysis process can be simplified.

According to the present embodiment, when blood coagulation reaction has not occurred, the measurement result is masked as shown in FIG. 9. Thus, the user can easily understand whether blood coagulation reaction occurred during the measurement, and thus, can advance the procedure thereafter smoothly. Moreover, when the button D 13 in FIG. 9 has been operated, contents of the error are displayed as shown in FIG. 10, and thus, the user can more easily understand whether blood coagulation reaction occurred.

In the above embodiment, whether blood coagulation reaction has occurred is determined based on absorbance. However, whether blood coagulation reaction has occurred may be determined based on the transmitted light amount. Since the absorbance is obtained through logarithmic conversion of the transmitted light amount as described above, in a case where blood coagulation reaction has occurred, the difference between the amounts of changes in the transmitted light amount also becomes large between the wavelengths. Therefore, by comparing a value corresponding to this difference with a threshold value, whether blood coagulation reaction has occurred can be determined.

<Second embodiment>

In the first embodiment above, when it has been determined that blood coagulation reaction had not occurred during the measurement time period T1, the measurement result is masked on the screen shown in FIG. 9. In the present embodiment, when it has been determined that blood coagulation reaction had not occurred during the measurement time period T1, information indicating necessity/unnecessity of re-measurement is further received. When the user has inputted an instruction to perform re-measurement, the blood coagulation analyzer 1 performs measurement again on the sample for which it has been determined that blood coagulation reaction had not occurred, and calculates a prothrombin time (PT). Here, a measurement time period T2 in the re-measurement is set to be longer than the measurement time period T1 in the measurement of the first time (for example, T2 = 2 x T1).

In the present embodiment, in order to allow re-measurement, the sample is dispensed from a sample container 101 by an extra amount into an empty cuvette 104 on the cuvette table 13. Then, at the time of the measurement of the first time, the sample is dispensed by the sample dispensing arm 17, from the cuvette 104 containing the sample on the cuvette table 13 into another empty cuvette 104 by a predetermined amount. At this time, in order to allow measurement of the second time, the remainder of the sample is left in the original cuvette 104. Then, with respect to the cuvette 104 into which the sample has been dispensed, measurement of the first time is performed through the same process as described in first embodiment. When it has been determined that blood coagulation reaction had not occurred as a result of this measurement, the sample is dispensed from the original cuvette 104 on the cuvette table 13 into an empty cuvette 104 again, and measurement is performed again with respect to this cuvette 104.

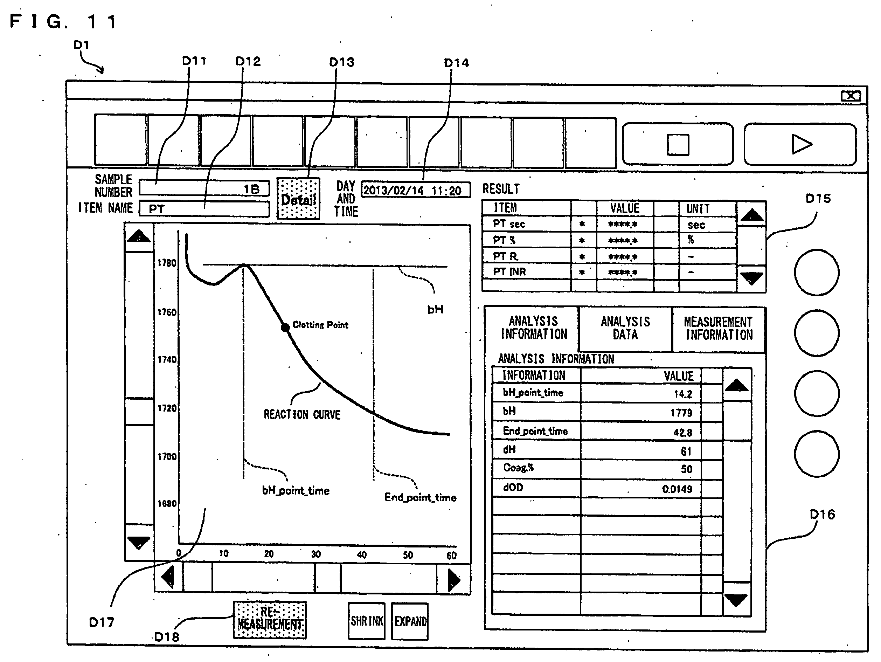

FIG. 11 shows a display screen showing a measurement result according to the present embodiment. To the screen D1 in FIG. 11, a button D18 as a reception section has been added, compared with the screen D1 in FIG. 9. When it has been determined that blood coagulation reaction had not occurred in the measurement of the first time, the button D18 is enabled and displayed in a predetermined color. By operating the button D18, the user can cause the blood coagulation analyzer 1 to perform re-measurement on the same sample. When it has been determined that blood coagulation reaction had occurred in the measurement of the first time, the button D18 is disabled.

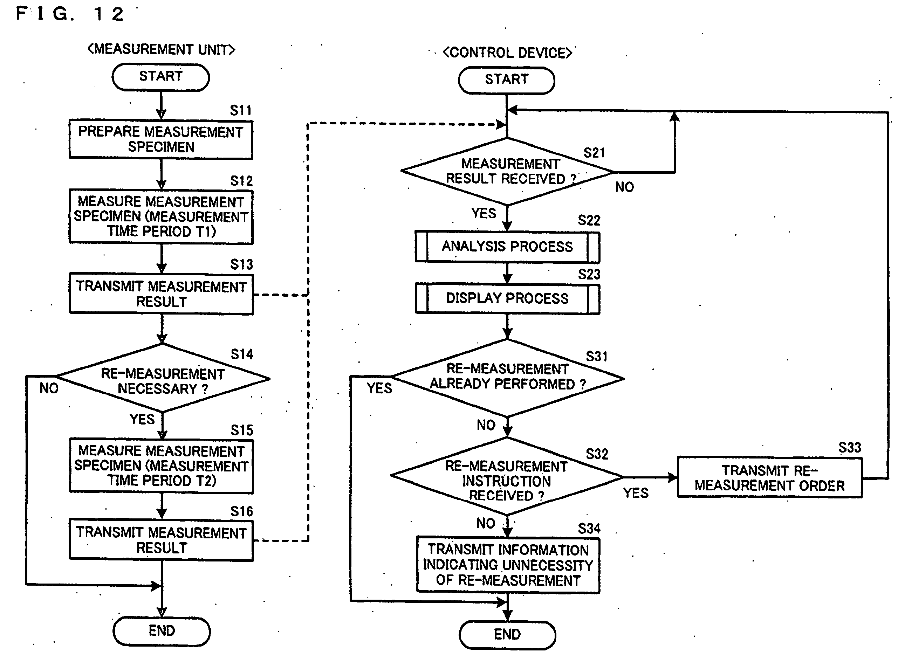

FIG. 12 is a flow chart showing a measurement process of a prothrombin time (PT) in the present embodiment. In steps S11 to S13 and S21 to S23 in FIG. 12, the same processes as those in the corresponding steps in FIG. 8A are performed.

In the measurement process of the first time, the measurement unit 2 performs the processes of steps S11 to S13, and transmits measurement results to the control device 4. Then, the measurement unit 2 waits until information indicating necessity/unnecessity of remeasurement is transmitted from the control device 4 (S14).

Upon receiving the measurement results of the first time from the measurement unit 2 (S21), the control device 4 performs the analysis process (S22), and further performs the display process based on the analysis result (S23). In a case where the flag (S106 in FIG. 8B) has been given in the analysis process (S22), the control device 4 masks the values of the measurement result in the screen D1 and enables the button D 18, as shown in FIG. 11. In a case where the flag has not been given, the control device 4 displays the measurement result in the region D15 and disables the button D18.

Subsequently, the control device 4 determines whether re-measurement has already been performed (S31). When re-measurement has not been performed (S31: NO), the control device 4 determines whether an instruction to perform re-measurement has been inputted by the user (S32). When the flag has not been given in the analysis process (S22), the determination in S32 becomes NO. Also when the button D18 has not been operated before the screen in FIG. 11 is closed, the determination in S32 becomes NO. When the determination in S32 is NO, the control device 4 transmits, to the measurement unit 2, information indicating that re-measurement is unnecessary (S34), and ends the processing. By receiving this information, the measurement unit 2 determines as NO in S14 and ends the processing.

When the button D18 has been operated and an instruction to perform re-measurement has been inputted by the user (S32: YES), the control device 4 transmits, to the measurement unit 2, information indicating that re-measurement is necessary (S33), and waits for measurement results to be transmitted from the measurement unit 2 (S21). By receiving this information from the control device 4, the measurement unit 2 determines as YES in S14 and performs again measurement on the same sample for which it has been determined that blood coagulation reaction had not occurred (S15). This measurement is performed for the measurement time period T2 which is longer than the measurement time period T1. When the re-measurement has ended, the measurement unit 2 transmits measurement results to the control device 4 (S16) and ends the processing.

Upon receiving the measurement results obtained through the re-measurement (S21), the control device 4 performs the analysis process on the received measurement results (S22), and further performs the display process based on an analysis result (S23). At this time, when blood coagulation reaction has occurred during the re-measurement, a measurement result is displayed in the region D15 on the screen D1. Subsequently, the control device 4 determines whether re-measurement has already been performed (S31). Since this measurement is re-measurement, the control device 4 determines as YES in S31 and ends the processing.

In the present embodiment, re-measurement is performed as appropriate for the measurement time period T2 which is longer than the measurement of the first time. Therefore, even with respect to a sample for which blood coagulation reaction did not occur during the measurement of the first time, there arises a possibility that blood coagulation reaction occurs during the re-measurement, and thus, the possibility that a blood coagulation time (PT) is appropriately obtained is increased.

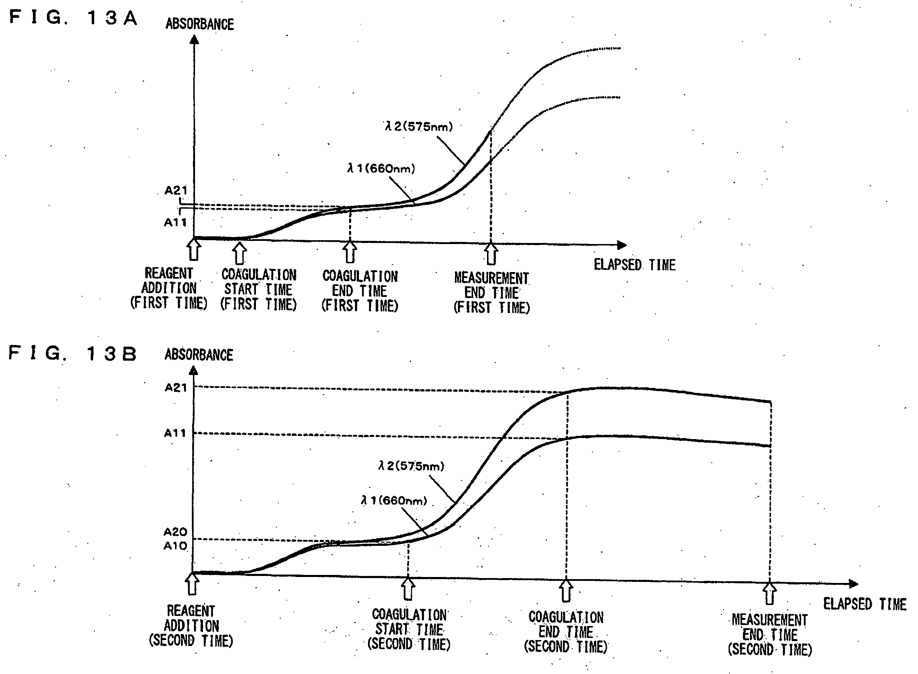

FIG. 13A and FIG. 13B show effects of the present embodiment. FIG. 13A schematically shows change in absorbance during measurement of the first time, and FIG. 13B schematically shows change in absorbance during re-measurement.

In the example in FIG. 13A, blood coagulation reaction starts at a timing a little before the measurement end time of the first time, and absorbance increases. However, in this case, since the timing when blood coagulation reaction is saturated is later than the measurement end time, a true measurement end time cannot be set, and thus, a coagulation point and a prothrombin time (PT) cannot be appropriately obtained. In the example in FIG. 13A, the coagulation start time and the coagulation end time are set based on the waveform which exists before blood coagulation reaction and which gently curves. However, this waveform is not caused by blood coagulation reaction, and thus, the difference between the absorbance based on the wavelength of 660 nm and the absorbance based on the wavelength of 575 nm at the coagulation end time is smaller than that caused by blood coagulation reaction. Therefore, it is determined that the coagulation end time set at the measurement of the first time is not true (blood coagulation reaction has not occurred), and a screen on which values of the measurement result are masked is displayed as shown in FIG. 11.

Then, when the user has inputted an instruction to perform re-measurement, re-measurement is performed on the same sample for the measurement time period T2 which is longer than in the measurement of the first time. In this case, as shown in FIG. 13B, the timing when blood coagulation reaction is saturated is earlier than the measurement end time. Thus, a true measurement end time can be set, and a coagulation point and a prothrombin time (PT) can be appropriately obtained. Further, since blood coagulation reaction has occurred during the measurement time period T2, the difference between the absorbance based on the wavelength of 660 nm and the absorbance based on the wavelength of 575 nm becomes large at the coagulation end time. Thus, it is determined that the coagulation end time set during the re-measurement is true, i.e., that blood coagulation reaction has occurred, and values of the measurement result are displayed in the region D15 in FIG. 11.

As described above, according to the present embodiment, the possibility that a prothrombin time is obtained through the re-measurement is increased. Therefore, an effect that an appropriate measurement result can be provided to the user can be exhibited.

<Third embodiment>

In the second embodiment, when it has been determined that blood coagulation reaction had not occurred, re-measurement is performed after waiting for receiving an instruction from the user. In contrast, in the present embodiment, when it has been determined that blood coagulation reaction had not occurred, re-measurement is automatically performed.

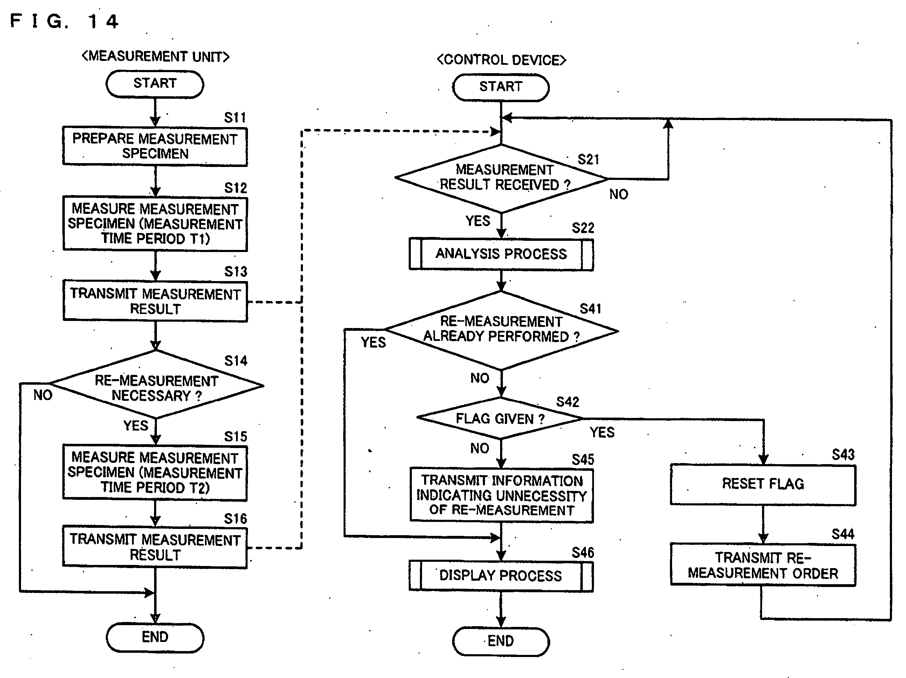

FIG. 14 is a flow chart showing a measurement process of a prothrombin time (PT) in the present embodiment. In steps S 11 to S 16 and steps S21 and S22 in FIG. 14, the same processes as those in the corresponding steps in FIG. 12 are performed.

Upon receiving measurement results of the first time from the measurement unit 2 (S21), the control device 4 performs the analysis process shown in FIG. 8B (S22). Subsequently, the control device 4 determines whether re-measurement has already been performed (S41). When re-measurement has not been performed (S41: NO), the control device 4 determines whether the flag has been given in the analysis process (S22) (S42). When the flag has not been given, the control device 4 transmits, to the measurement unit 2, information indicating that re-measurement is unnecessary (S45). By receiving this information, the measurement unit 2 determines as NO in S14 and ends the processing. Further, the control device 4 performs the display process based on the information obtained through the analysis process (S22) (S46). Here, since the flag has not been given, values of the measurement result are displayed in the region D15 in FIG. 9. It should be noted that, on the screen displayed in S46, the button D18 shown in FIG. 11 is not included.

When the flag has been given in the analysis process (S22) (S42: YES), the control device 4 resets the flag (S43), further transmits, to the measurement unit 2, information indicating that re-measurement is necessary (S44), and waits for measurement results to be transmitted from the measurement unit 2 (S21). By receiving this information, the measurement unit 2 determines as YES in S14 and performs measurement again on the same sample for which it has been determined that blood coagulation reaction had not occurred (S 15). This measurement is performed for the measurement time period T2 which is longer than the measurement time period T1, as in the second embodiment. When the re-measurement has ended, the measurement unit 2 transmits measurement results to the control device 4 (S16) and ends the processing.

Upon receiving the measurement results obtained through the re-measurement (S21), the control device 4 performs the analysis process on the received measurement results (S22), and further determines whether re-measurement has already been performed (S41). Since this measurement is re-measurement, the control device 4 determines as YES in S41 and performs the display process (S46). At this time, when blood coagulation reaction has occurred during the re-measurement, a measurement result is displayed in the region D15 on the screen D1. Further, when blood coagulation reaction has not occurred even during the re-measurement, the measurement result is masked in the region D15 on the screen D 1. After performing the display process in this manner, the control device 4 ends the processing.

Also in the present embodiment, as in the second embodiment, re-measurement is performed for the measurement time period T2 which is longer than in the measurement of the first time. Thus, the possibility that a prothrombin time is obtained through the re-measurement is increased. Therefore, an effect that an appropriate measurement result can be provided to the user can be exhibited. Further, according to the present embodiment, when it has been determined that blood coagulation reaction had not occurred, re-measurement is automatically performed. Thus, the process of re-measurement can be more smoothly advanced.

In the present embodiment, when it has been determined that blood coagulation reaction had not occurred during the measurement of the first time, the screen showing the measurement result is not displayed, and after the re-measurement, the screen showing the measurement result is displayed. Thus, the user cannot know whether the displayed measurement result was obtained through the measurement of the first time or through the re-measurement.

Therefore, it is preferable that the screen displayed in S46 includes information indicating whether the measurement result was obtained through the measurement of the first time or through the re-measurement.

<Fourth embodiment>

In the first to third embodiments above, when the measurement time period T1 has elapsed, the measurement unit 2 stops measurement on the measurement specimen. In contrast, in the present embodiment, until the control device 4 has determined that the coagulation end time is based on blood coagulation reaction, measurement on the measurement specimen is continued.

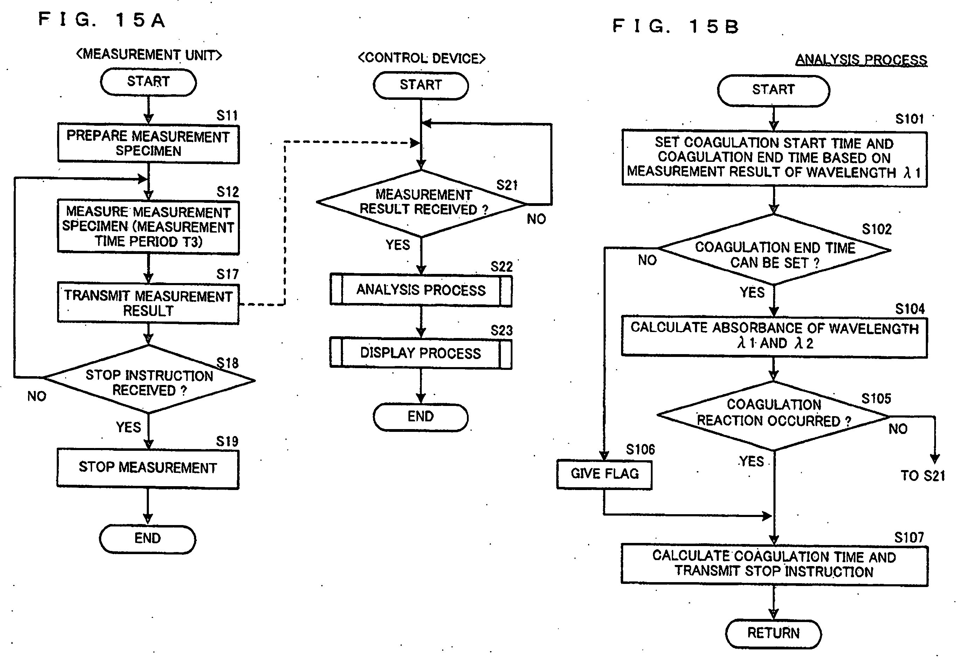

FIG. 15A and FIG. 15B are flow charts showing a measurement process of a prothrombin time (PT) in the present embodiment. In step S11, step S12, steps S21 to S23, and step S101, step S102, steps S104 to S106 in FIG. 15, the same processes as those in the corresponding steps in FIG. 8 are performed.

with reference to FIG. 15A, the measurement unit 2 prepares a measurement specimen (S11) and measures the measurement specimen (S12). When a measurement time period T3 has elapsed, the measurement unit 2 transmits, to the control device 4, data being measurement results based on the above two wavelengths and being stored in the memory 202, without stopping measurement on the measurement specimen (S17). The measurement time period T3 is set as appropriate in accordance with the present embodiment. Until receiving an instruction to stop measurement from the control device 4 (S18: NO), the measurement unit 2 continues transmitting data being measurement results to the control device 4, every time the measurement time period T3 has elapsed (S17).

Upon receiving the data being measurement results from the measurement unit 2 (S21: YES), the control device 4 performs the analysis process on the received measurement results, and calculates a prothrombin time (PT) of the measurement specimen (S22). With reference to FIG. 15B, in the analysis process in S22, the control device 4 sets a coagulation start time and a coagulation end time based on the transmitted light amount being data based on light of 660 nm wavelength, among the received measurement results (S101), and determines whether a coagulation end time has been able to be set (S102). When a coagulation end time has failed to be set (S102: NO), the control device 4 causes the memory 202 to hold the flag indicating that blood coagulation reaction has not occurred (S106). When a coagulation end time has been able to be set (S102: YES), the control device 4 calculates an absorbance A10 at the coagulation start time and an absorbance A11 at the coagulation end time with respect to the transmitted light amount being data based on light of 660 nm wavelength, and further, calculates an absorbance A20 at the coagulation start time and an absorbance A21 at the coagulation end time with respect to the transmitted light amount being data based on light of 575 nm wavelength (S104). The control device 4 determines whether the coagulation end time set in S101 is based on blood coagulation reaction, based on formula (4) above (S105). Upon determining that the set coagulation end time is not based on blood coagulation reaction (S105: NO), the control device 4 returns to S21 and waits until receiving data being the measurement results from the measurement unit 2. On the other hand, upon determining that the coagulation end time set in S101 is based on blood coagulation reaction (S105: YES), the control device 4 sets a coagulation point based on the coagulation end time, calculates a blood coagulation time (prothrombin time), and transmits an instruction to stop measurement to the measurement unit 2 (S107), and ends the analysis process. With reference back to FIG. 15A, after performing the analysis process (S22), the control device 4 performs the display process of the analysis result (S23).

Upon receiving an instruction to stop measurement from the control device 4 (S18: YES), the measurement unit 2 stops measurement on the measurement specimen (S19).

According to the present embodiment, when it has been determined that blood coagulation reaction had not occurred, measurement on the measurement specimen is continued, and when it has been determined that blood coagulation reaction had occurred, measurement on the measurement specimen is stopped. Therefore, the possibility that an accurate prothrombin time is obtained through one measurement is increased. Therefore, an effect that an appropriate measurement result can be quickly and assuredly provided to the user can be exhibited.

<Modification>

The first to fourth embodiments have been described above. However, the present invention is not limited to these embodiments in any way. Other than the above, various modifications can be made to the embodiments of the present invention.

For example, in the first to fourth embodiments, the ratio between the amount of change in absorbance at the wavelength of 660 nm and the amount of change in absorbance at the wavelength of 575 nm is compared with a predetermined threshold value, whereby whether blood coagulation reaction has occurred is determined. However, the determination method of whether blood coagulation reaction has occurred is not limited thereto. Another determination method based on optical information of lights having different wavelengths may be used. For example, the difference between the amount of change in absorbance at the wavelength of 660 nm and the amount of change in absorbance at the wavelength of 575 nm is compared with a predetermined threshold value, whereby whether blood coagulation reaction has occurred may be determined. In this case, determination in step 5105 in FIG. 8B is performed based on the following determination condition. In formula (5), Ash' is a threshold value:

$$(A21 - A20) - (A11 - A10) \geq Ash' \quad ...(5)$$

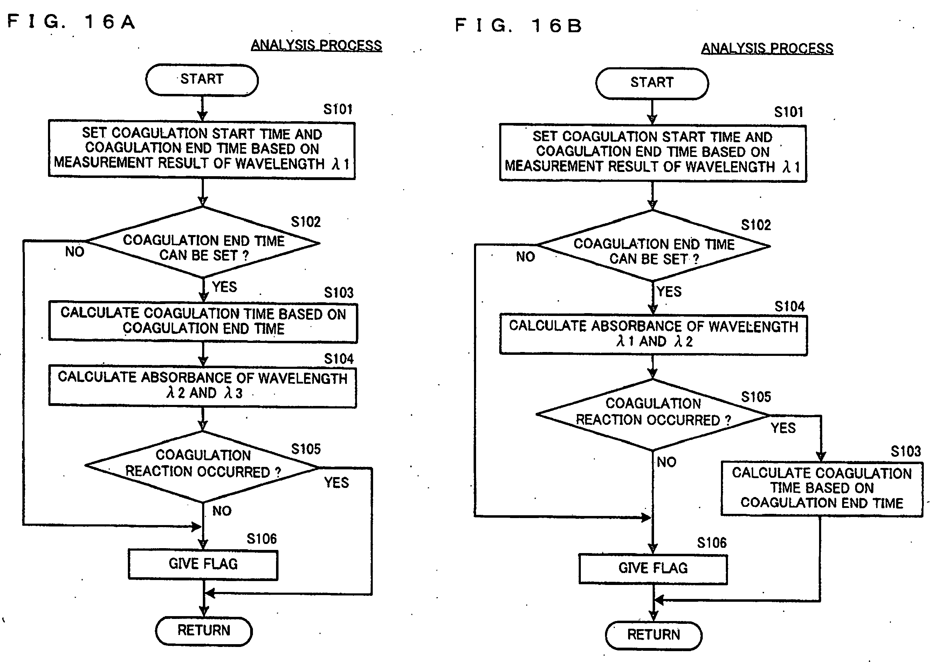

In the first to fourth embodiments, as shown in FIG. 8B, after a prothrombin time (PT) has been calculated in step S103, whether blood coagulation reaction has occurred is determined in steps S104 and S105. However, the timing at which a prothrombin time (PT) is calculated is not limited thereto, and another timing may be used. For example, as shown in FIG. 16B, when it has been determined that blood coagulation reaction had occurred in steps S104 and S105, step S103 is performed, and a prothrombin time (PT) may be calculated.

In the first to fourth embodiments, by using transmitted light that transmits through the measurement specimen, calculation of a prothrombin time and determination of whether blood coagulation reaction has occurred are performed. However, by using scattered light that is scattered by the measurement specimen, calculation of a prothrombin time and determination of whether blood coagulation reaction has occurred may be performed.

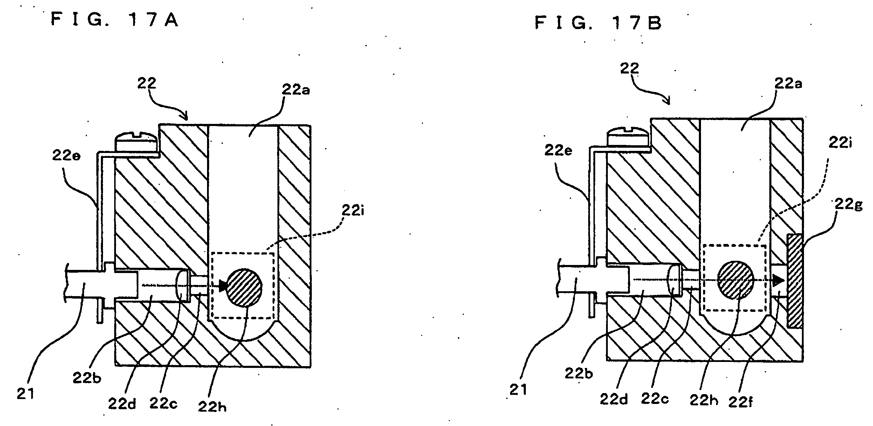

FIG. 17A shows a structure of the detection part 22 when scattered light is used. In this structure example, a hole 22h is provided in an inner surface of the holder 22a, at a position at the same level as the communication hole 22c, and a light detector 22i is arranged at the back of the hole 22h. When a cuvette 104 is inserted in the holder 22a, and light is emitted from the optical fiber 21, light scattered by the measurement specimen in the cuvette 104 enters, via the hole 22h, the light detector 22i as a light receiving part.

In this structure example, a detection signal from the light detector 22i represents a scattered light intensity by the measurement specimen. As shown in formula (3) above, a scattered light intensity is inversely proportional to the fourth power of the wavelength. Therefore, when blood coagulation reaction has occurred, the difference between the amounts of changes in scattered light intensities regarding the lights of two wavelengths becomes large. Thus, as in the first to fourth embodiments, by comparing a value representing this difference with a predetermined threshold value, whether blood coagulation reaction has occurred can be determined. Also in the structure example in FIG. 17A, whether blood coagulation reaction has occurred can be appropriately determined, based on detection signals based on lights of two wavelengths outputted from the light detector 22i.

As shown in FIG. 17B, it may be configured such that both of transmitted light that transmits through the measurement specimen and scattered light that is scattered by the measurement specimen can be detected. In this case, for example, by using either one of detection signals respectively outputted from the light detectors 22g and 22i, calculation of a prothrombin time is performed, and by using the other one, whether blood coagulation reaction has occurred is determined.

In the first to fourth embodiments, by the measurement result being masked on the screen D1, the user is notified that a prothrombin time cannot be appropriately calculated, i.e., blood coagulation reaction did not occur. However, the notification method is not limited thereto. For example, the measurement result is displayed and also an indication for making notification that a prothrombin time cannot be appropriately calculated may be included in the screen D1. Alternatively, a sound indicating that a prothrombin time cannot be appropriately calculated may be outputted.

In the second and third embodiments, the number of times of re-measurements is one. However, the re-measurement may be performed two or more times. In this case, preferably, as the number of times of the re-measurements is increased, the measurement time period is accordingly extended.

Further, the present invention can be applied as appropriate to measurement and analysis of items regarding blood coagulation other than a prothrombin time.

Various modifications can be made as appropriate to the embodiments of the present invention without departing from the scope of the technical idea defined by the claims.

**Claims**

**1.** A blood coagulation analyzer comprising:

an obtaining section (20, 22, 216) configured to irradiate a measurement specimen prepared by mixing a blood specimen and a reagent together, the obtaining section (20, 22, 216) comprising:

a light source (20) configured to emit light of
a first wavelength and light of a second wavelength which is different from the first wavelength to the measurement specimen; and

a light receiving part (22) configured to receive lights of the first and second wavelengths from the measurement specimen, the obtaining section (20, 22, 216) configured to obtain, from the measurement specimen, first and second optical information based on the lights of the first and second wavelengths, respectively; and

a controller (4) configured for performing operations comprising:

calculating a blood coagulation time based on the first optical information;
wherein
the controller (4) is configured to calculate an amount of change of the light of the first wavelength and the amount of change of the light of the second wavelength, based on the first optical information and the second optical information, respectively;
**characterised in that**: the controller (4) is configured to specify a start time and an end time of a blood coagulation reaction based on the predetermined optical information, and is configured to calculate the blood coagulation time based on the start time and the end time which have been specified; **in that**:

the controller (4) is configured to calculate the amount of change of the light of the first wavelength and the amount of change of the light of the second wavelength, based on the first and second optical information, respectively, in a period from the start time to the end time; and **in that**:
the controller (4) is configured to determine whether a relation between the amount of change of the first optical information and the amount of change of the second optical information satisfies a predetermined condition, based on a comparison of a ratio or a difference between the amount of change of the first optical information and the amount of change of the second optical information, with a predetermined threshold value.

2. The blood coagulation analyzer according to claim 1, wherein

the obtaining section (20, 22, 216) is configured to detect at least one of an intensity of light that has transmitted through the measurement specimen and an intensity of light that has been scattered by the measurement specimen.

3. The blood coagulation analyzer according to claim 1, wherein

the controller (4) is configured to determine appropriateness/inappropriateness of the calculated end time, by determining whether the relation between the amount of change of the light of the first wavelength and the amount of change of the light of the second wavelength satisfies a predetermined condition.

4. The blood coagulation analyzer according to any one of claims 1 to 3, wherein

the controller (4) is configured to calculate a coagulation time based on the optical information based on the light of the first wavelength and the optical information based on the light of the second wavelength.

5. The blood coagulation analyzer according to any one of claims 1 to 4, wherein

the obtaining section (20, 22, 216) is configured to at least obtain an absorbance based on the light of the first wavelength and an absorbance based on the light of the second wavelength, as the optical information based on the light of the first wavelength and the optical information based on the light of the second wavelength, and the controller (4) is configured to determine whether the relation between the amount of change of the light of the first wavelength and the amount of change of the light of the second wavelength satisfies a predetermined condition, based on the absorbance based on the light of the first wavelength and the absorbance based on the light of the second wavelength.

6. The blood coagulation analyzer according to any one of claims 1 to 5, further comprising:

a notification section (41) configured to make notification, when the controller (4) has determined that the relation between the amount of change of the light of the first wavelength and the amount of change of the light of the second wavelength does not satisfy a predetermined condition, that a blood coagulation time cannot be appropriately obtained.

7. The blood coagulation analyzer according to any one of claims 1 to 6, further comprising:

a reception section (D18) for receiving an instruction to perform re-measurement of a blood coagulation time, when the controller (4) has determined that the relation between the amount of change of the light of the first wavelength and the amount of change of the light of the second wavelength does not satisfy the predetermined condition, wherein

upon the reception section (D18) receiving the instruction, the controller (4) is configured to cause the obtaining section (20, 22, 216) to perform re-measurement of a blood coagulation time, on a measurement specimen for re-measurement prepared by mixing a reagent to a blood specimen identical to the blood specimen for which it has been determined that the relation between the amount of change of the light of the first wavelength and the amount of change of the light of the second wavelength does not satisfy the predetermined condition.

8. The blood coagulation analyzer according to claim 7, wherein

the obtaining section (20, 22, 216) is configured to obtain the optical information, with a measurement time period for re-measurement set to be longer than an ordinary measurement time period.

9. A blood coagulation analyzing method comprising:

irradiating a measurement specimen prepared by mixing a blood specimen and a reagent together;
emitting light of a first wavelength and light of a second wavelength which is different from the first wavelength to the measurement specimen;
receiving lights of the first and second wavelengths from the measurement specimen to obtain, from the measurement specimen, first and second optical information based on the lights of the first and second wavelengths, respectively; and
controlling the performance operations, the controlling comprising:

calculating a blood coagulation time based on the first optical information;
calculating an amount of change of the light of the first wavelength and the amount of change of the light of the second wavelength, based on the first optical information and the second optical information, respectively;
**characterised by**: specifing a start time and an end time of a blood coagulation reaction based on the predetermined optical information, and calculating the blood coagulation time based on the start time and the end time which have been specified; by:

calculating the amount of change of the light of the first wavelength and the amount of change of the light of the second wavelength, based on the first and second optical information, respectively, in a period from the start time to the end time; and by:

determining whether a relation between the amount of change of the first optical information and the amount of change of the second optical information satisfies a predetermined condition, based on a comparison of a ratio or a difference between the amount of change of the first optical information and the amount of change of the second optical information, with a predetermined threshold value.

**Patentansprüche**

1. Blutgerinnungsanalysator, mit:

einem Erfassungsabschnitt (20, 22, 216), der ausgestaltet ist, um eine Messprobe, die durch Mischen einer Blutprobe und einer Reagenz miteinander separiert worden ist, bestrahlt, wobei der Erfassungsabschnitt (20, 22, 216) aufweist:

eine Lichtquelle (20), die ausgestaltet ist Licht einer ersten Wellenlänge und Licht einer zweiten Wellenlänge, die sich von der ersten Wellenlänge unterscheidet, zu der Messprobe auszusenden; und

ein Lichtempfangsteil (22), der ausgestaltet ist mit der ersten und zweiten Wellenlänge von der Messprobe zu empfangen, wobei der Erfassungsabschnitt (20, 22, 216) ausgestaltet ist, um von der Messprobe erste und zweite optische Informationen auf Grundlage des Lichts der ersten bzw. zweiten Wellenlänge zu erhalten; und

eine Steuereinrichtung (4), die ausgestaltet ist zum Ausführen von Operationen mit:

Berechnen einer Blutgerinnungszeit auf Grundlage der ersten optischen Information;
wobei
die Steuereinrichtung (4) einen Änderungsbetrag des Lichts der ersten Wellenlänge und den Änderungsbetrag des Lichts der zweiten Wellenlänge auf Grundlage der ersten optischen Information bzw. der zweiten optischen Information, berechnet;
**dadurch gekennzeichnet, dass**: die Steuereinrichtung (4) ausgestaltet ist, um eine Startzeit und eine Endzeit einer Blutgerinnungsreaktion basierend auf den vorbestimmten optischen Informationen zu spezifizieren und ausgestaltet ist, um die Blutgerinnungszeit und basierend auf der Startzeit und der Endzeit, welche spezifiziert worden sind, zu berechnen; dadurch dass:

die Steuereinrichtung (4) ausgestaltet ist, um den Änderungsbetrag des Lichts der ersten Wellenlänge und den Änderungsbetrag des Lichts der zweiten Wellenlänge basierend auf den ersten bzw. zweiten optischen Informationen in einem Zeitraum von der Startzeit zu der Endzeit zu berechnen; und dadurch dass:

die Steuereinrichtung (4) ausgestaltet ist, um zu bestimmen ob eine Beziehung zwischen dem Änderungsbetrag der ersten optischen Information und des Änderungsbetrags der zweiten optischen Information eine vorbestimmte Bedingung erfüllt, basierend auf einem Vergleich eines Verhältnisses oder einer Differenz zwischen dem Änderungsbetrag der ersten optischen Information und des Änderungsbetrags der zweiten optischen Information mit einem vorbestimmten Schwellwert.

2. Blutgerinnungsanalysator nach Anspruch 1, wobei
der Erfassungsabschnitt (20, 22, 216) ausgestaltet ist, um eine Intensität von Licht, das durch die Messprobe gesendet worden ist und/oder Intensität von Licht, das durch die Messprobe gestreut worden ist, erfasst.

3. Blutgerinnungsanalysator nach Anspruch 1, wobei die Steuereinheit ausgestaltet ist, um die Angemessenheit/Unangemessenheit der berechneten Endzeit bestimmt durch Bestimmen ob die Beziehung zwischen dem Änderungsbetrag des Lichts der ersten Wellenlänge und des Änderungsbetrags des Lichts der zweiten Wellenlänge eine vorbestimmte Bedingung erfüllt.

4. Blutgerinnungsanalysator nach einem der Ansprüche 1 bis 3, wobei
die Steuereinrichtung (4) ausgestaltet ist, um eine Gerinnungszeit basierend auf den optischen Informationen, die auf dem Licht der ersten Wellenlänge basieren und den optischen Informationen, die auf dem Licht der zweiten Länge basieren, zu berechnen.

5. Blutgerinnungsanalysator nach einem der Ansprüche 1 bis 4, wobei
der Erfassungsabschnitt (20, 22, 216) ausgestaltet ist, um zumindest ein Absorptionsvermögen basierend auf dem Licht der ersten Wellenlänge und ein Absorptionsvermögen basierend auf dem Licht der zweiten Wellenlänge als die optische Information basierend auf dem Licht der ersten Wellenlänge und den optischen Informationen basierend auf dem Licht der zweiten Wellenlänge zu erhalten, und
die Steuereinrichtung (4) ausgestaltet ist, um zu bestimmen, ob die Beziehung zwischen dem Änderungsbetrag des Lichts der ersten Wellenlänge und des Änderungsbetrags des Lichts der zweiten Wellenlänge eine vorbestimmte Bedingung erfüllt basierend auf dem Absorptionsvermögen basierend auf dem Licht der ersten Wellenlänge und dem Absorptionsvermögen basierend auf dem Licht der zweiten Wellenlänge.

6. Blutgerinnungsanalysator nach einem der Ansprüche 1 bis 5, ferner mit:

einem Benachrichtigungsabschnitt (41), der ausgestaltet ist, um eine Benachrichtigung zu erzeugen, wenn die Steuereinrichtung (4) bestimmt hat, dass die Beziehung zwischen dem Änderungsbetrag des Lichts der ersten Wellenlänge und dem Änderungsbetrag des Lichts der zweiten Wellenlänge nicht die vorbestimmte Bedingung erfüllt, dass eine Blutgerinnungszeit nicht angemessen erhalten werden kann.

7. Blutgerinnungsanalysator nach einem der Ansprüche 1 bis 6, ferner mit:

einem Empfangsabschnitt (D18) zum Empfangen einer Anweisung zum Durchführen einer Nachmessung einer Blutgerinnungszeit, wenn die Steuereinrichtung (4) bestimmt hat, dass die Beziehung zwischen dem Änderungsbetrag des Lichts der ersten Wellenlänge und dem Änderungsbetrag des Lichts der zweiten Wellenlänge nicht die vorbestimmte Bedingung erfüllt, wobei
mit Empfangen der Anweisung durch den Empfangsabschnitt (D18) die Steuereinheit (4) ausgestaltet ist um zu bewirken, dass der Erfassungsabschnitt (20, 22, 216) eine Nachmessung einer Blutgerinnungszeit an einer Messprobe zum Nachmessen präpariert durch Mischen einer Reagenz mit einer Blutprobe identisch zu der Blutprobe für welche bestimmten wurde, dass die Beziehung zwischen dem Änderungsbetrag des Lichts der ersten Wellenlänge und des Änderungsbetrags des Lichts der zweiten Wellenlänge nicht die vorbestimmte Bedingung erfüllt, durchführt.

8. Blutgerinnungsanalysator nach Anspruch 7, wobei
der Erfassungsabschnitt (20, 22, 216) ausgestaltet ist, um die optische Information mit einer Messzeitspanne zur Nachmessung, die länger eingestellt ist, als eine normale Messzeitspanne, erhält.

9. Verfahren zur Blutgerinnungsanalyse , mit:

Bestrahlen einer Messprobe, die durch Mischen einer Blutprobe in einem Reagenz präpariert worden ist;
Emittieren von Licht einer ersten Wellenlänge und Licht einer zweiten Wellenlänge, die von der ersten Wellenlänge verschieden ist auf die Messprobe;
Empfangen von Licht der ersten und zweiten Wellenlänge von der Messprobe, um von der Messprobe erste und zweite optische Informationen basierend auf dem Licht der ersten bzw. zweiten Wellenlänge zu erhalten; und Steuern der Leistungsoperationen, wobei das Steuern aufweist:

Berechnen einer Blutgerinnungszeit basierend auf den ersten optischen Informationen; Berechnen eines Änderungsbetrags des Lichts der ersten Wellenlänge und des Änderungsbetrags des Lichts der zweiten Wellenlänge basierend auf der ersten optischen Information bzw. der zweiten optischen Information;

**gekennzeichnet, durch**:

Spezifizieren einer Startzeit und einer Endzeit einer Blutgerinnungsreaktion basierend auf den vorbestimmten optischen Informationen und Berechnen der Blutgerinnungszeit basierend auf der Anfangszeit und der Endzeit, die spezifiziert worden sind; **durch**:

Berechnen des Änderungsbetrags des Lichts der ersten Wellenlänge und des Änderungsbetrags des Lichts der zweiten Wellenlänge basierend auf der ersten bzw. zweiten optischen Information in einem Zeitraum von der Startzeit zu der Endzeit, und **durch**
Bestimmen ob eine Beziehung zwischen dem Änderungsbetrag der ersten optischen Information und dem Änderungsbetrag der zweiten optischen Information eine vorbestimmte Bedingung erfüllt basierend auf einem Vergleich eines Verhältnisses oder einer Differenz zwischen dem Änderungsbetrag der ersten optischen Information und dem Änderungsbetrag der zweiten optischen Information mit einem vorbestimmten Schwellwert.

## Revendications

1. Analyseur de coagulation sanguine, comprenant:

une section d'obtention (20, 22, 216) conçue pour irradier un échantillon de mesure préparé par mélange conjoint d'un échantillon de sang et d'un réactif , la section d'obtention (20, 22, 216) comprenant:

une source de lumière (20) conçue pour émettre de la lumière d'une première longueur d'onde et de la lumière d'une seconde longueur d'onde qui est différente de la première longueur d'onde sur l'échantillon de mesure; et

une partie de réception de lumière (22) conçue pour recevoir les lumières des première et seconde longueurs d'onde de l'échantillon de mesure, la section d'obtention (20, 22, 216) conçue pour obtenir, de l'échantillon de mesure, des premières et secondes informations optiques basées sur les lumières des première et seconde longueurs d'onde, respectivement ; et

un organe de commande (4) conçu pour réaliser des opérations comprenant :

le calcul d'un temps de coagulation sanguine sur la base des premières informations optiques ;

dans lequel :

l'organe de commande (4) est conçu pour calculer une quantité de changement de la lumière de la première longueur d'onde et la quantité de changement de la lumière de la seconde longueur d'onde, sur la base des premières informations optiques et des secondes informations optiques, respectivement ;

**caractérisé en ce que** : l'organe de commande (4) est conçu pour spécifier un moment de début et un moment de fin d'une réaction de coagulation sanguine sur la base des informations optiques prédéterminées et est conçu pour calculer le temps de coagulation sanguine sur la base du moment de début et du moment de fin qui ont été spécifiés ; **en ce que** :

l'organe de commande (4) est conçu pour calculer la quantité de changement de la lumière de la première longueur d'onde et la quantité de changement de la lumière de la seconde longueur d'onde, sur la base des premières et secondes informations optiques, respectivement, au cours d'une période allant du moment de début au moment de fin ; et **en ce que** :

l'organe de commande (4) est conçu pour déterminer si une relation entre la quantité de changement des premières informations optiques et la quantité de changement des secondes informations optiques satisfait à une condition prédéterminée sur la base d'une comparaison d'un rapport ou d'une différence entre la quantité de changement des premières informations optiques et la quantité de changement des secondes informations optiques, avec une valeur de seuil prédéterminée.

2. Analyseur de coagulation sanguine selon la revendication 1, dans lequel :

la section d'obtention (20, 22, 216) est conçue pour détecter au moins l'une d'une intensité de lumière qui a été transmise par l'échantillon de mesure et d'une intensité de lumière qui a été diffusée par l'échantillon de mesure.

3. Analyseur de coagulation sanguine selon la revendication 1, dans lequel :

l'organe de commande (4) est conçu pour déterminer l'adéquation/inadéquation du moment de fin calculé en déterminant si la relation entre la quantité de changement de la lumière de la première longueur d'onde et la quantité de changement de la lumière de la seconde longueur d'onde satisfait à une condition prédéterminée.

4. Analyseur de coagulation sanguine selon l'une quelconque des revendications 1 à 3, dans lequel l'organe de commande (4) est conçu pour calculer un temps de coagulation sur la base des informations optiques basées sur la lumière de la première longueur d'onde et des informations optiques basées sur la lumière de la seconde longueur d'onde.

5. Analyseur de coagulation sanguine selon l'une quelconque des revendications 1 à 4, dans lequel :

la section d'obtention (20, 22, 216) est conçue pour au moins obtenir une absorbance sur la base de la lumière de la première longueur d'onde et une absorbance sur la base de la lumière de la seconde longueur d'onde en tant qu'informations optiques basées sur la lumière de la première longueur d'onde et informations optiques basées sur la seconde longueur d'onde, et
l'organe de commande (4) est conçu pour déterminer si la relation entre la quantité de changement de la lumière de la première longueur d'onde et la quantité de changement de la lumière de la seconde longueur d'onde satisfait à une condition prédéterminée sur la base de l'absorbance basée sur la lumière de la première longueur d'onde et de l'absorbance basée sur la lumière de la seconde longueur d'onde.

**6.** Analyseur de coagulation sanguine selon l'une quelconque des revendications 1 à 5, comprenant en outre:

une section de notification (41) conçue pour notifier, lorsque l'organe de commande (4) a déterminé que la relation entre la quantité de changement de la lumière de la première longueur d'onde et la quantité de changement de la lumière de la seconde longueur d'onde ne satisfait pas à une condition prédéterminée, qu'un temps de coagulation sanguine ne peut pas être obtenu de manière appropriée .

**7.** Analyseur de coagulation sanguine selon l'une quelconque des revendications 1 à 6, comprenant en outre:

une section de réception (D18) pour la réception d'une instruction pour réaliser une nouvelle mesure d'un temps de coagulation sanguine lorsque l'organe de commande (4) a déterminé que la relation entre la quantité de changement de la lumière de la première longueur d'onde et la quantité de changement de la lumière de la seconde longueur d'onde ne satisfait pas à la condition prédéterminée, dans lequel,
lors de la réception de l'instruction par la section de réception (D18), l'organe de commande (4) est conçu pour entraîner la réalisation d'une nouvelle mesure d'un temps de coagulation sanguine par la section d'obtention (20, 22, 216), sur un échantillon de mesure destiné à une nouvelle mesure préparé par mélange d'un réactif à un échantillon de sang identique à l'échantillon de sang pour lequel il a été déterminé que la relation entre la quantité de changement de la lumière de la première longueur d'onde et la quantité de changement de. la lumière de la seconde longueur d'onde ne satisfait pas à la condition prédéterminée.

**8.** Analyseur de coagulation sanguine selon la revendication 7, dans lequel:

la section d'obtention (20, 22, 216) est conçue pour obtenir les informations optiques avec une durée de mesure pour la nouvelle mesure définie pour être plus longue qu'une durée de mesure ordinaire.

**9.** Procédé d'analyse de coagulation sanguine, comprenant:

l'irradiation d'un échantillon de mesure préparé par mélange conjoint d'un échantillon de sang et d'un réactif , l'émission de lumière d'une première longueur d'onde et de lumière d'une seconde longueur d'onde qui est différente de la première longueur d'onde sur l'échantillon de mesure ;
la réception de lumières des première et seconde longueurs d'onde de l'échantillon de mesure pour obtenir, de l'échantillon de mesure, des premières et secondes informations optiques sur la base des lumières des première et seconde longueurs d'onde, respectivement ; et
la commande des opérations de réalisation, la commande comprenant:

le calcul d'un temps de coagulation sanguine basé sur les premières informations optiques ;
le calcul d'une quantité de changement de la lumière de la première longueur d'onde et de la quantité de changement de la lumière de la seconde longueur d'onde sur la base des premières informations optiques et des secondes informations optiques, respectivement ;
**caractérisé par**: la spécification d'un temps de début et d'un temps de fin d'une réaction de coagulation sanguine sur la base des informations optiques prédéterminées et le calcul du temps de coagulation sanguine sur la base du moment de début et du moment de fin qui ont été spécifiés ;
en calculant la quantité de changement de la lumière de la première longueur d'onde et la quantité de changement de la lumière de la seconde longueur d'onde sur la base des premières et secondes informations optiques, respectivement, au cours d'une période allant du moment de début au moment de fin ; et
en déterminant si une relation entre la quantité de changement des premières informations optiques et la quantité de changement des secondes informations optiques satisfait ou non à une condition prédéterminée sur la base d'une comparaison d'un rapport ou d'une différence entre la quantité de changement des premières informations optiques et la quantité de changement des secondes informations optiques, avec une valeur de seuil prédéterminée.

FIG. 1

F I G. 2

F I G. 3 A

F I G. 3 B

F I G. 3 C

FIG. 4A

FIG. 4B

F I G. 5

STEPPING MOTOR SECTION ~211

ROTARY ENCODER SECTION ~212

LIQUID SURFACE DETECTION SENSOR ~213

SENSOR SECTION ~214

MECHANISM SECTION ~215

OBTAINING SECTION ~216

BAR CODE READER ~14

LAMP UNIT ~20

MEMORY 202

CPU 201

I/O 204

200

COMMUNICATION INTERFACE 203

TO SAMPLE TRANSPORTER 3

TO CONTROL DEVICE 4

2

EP 2 775 292 B1

27

F I G. 6

FIG. 7A

TRANSMITTED
LIGHT AMOUNT

VO

Vs

$Vc=Vs+(Ve-Vs)/k$

Vc

Ve

$\lambda 1$(660nm)

MEASUREMENT
START

COAGULATION
START TIME

COAGULATION
POINT

COAGULATION
END TIME

ELAPSED
TIME

FIG. 7B

ABSORBANCE

$\lambda 2$(575nm)

A2

$\lambda 1$(660nm)

A1

A0

MEASUREMENT
START

COAGULATION
START TIME

COAGULATION
POINT

COAGULATION
END TIME

MEASUREMENT
END TIME

ELAPSED
TIME

EP 2 775 292 B1

# FIG. 8A

<CONTROL DEVICE>

<MEASUREMENT UNIT>

```
        START                          START

          │ S11                          │
┌─────────────────────┐                  │◄──────────┐
│ PREPARE MEASUREMENT │                  ▼           │
│     SPECIMEN        │            ╱ MEASUREMENT ╲ S21│
└─────────────────────┘           ╱  RESULT      ╲   │
          │ S12                   ╲  RECEIVED ?   ╱───┘ NO
┌─────────────────────┐            ╲             ╱
│ MEASURE MEASUREMENT │                 │ YES
│ SPECIMEN (MEASUREMENT              ┌──────────────┐ S22
│  TIME PERIOD T1)    │             ║ ANALYSIS     ║
└─────────────────────┘             ║ PROCESS      ║
          │ S13                      └──────────────┘
┌─────────────────────┐                   │ S23
│ TRANSMIT MEASUREMENT│             ┌──────────────┐
│     RESULT          │- - - - - - ▶║ DISPLAY      ║
└─────────────────────┘             ║ PROCESS      ║
          │                          └──────────────┘
        END                               │
                                        END
```

# FIG. 8B

ANALYSIS PROCESS

```
                    START

                      │                        S101
      ┌──────────────────────────────────────┐
      │ SET COAGULATION START TIME AND        │
      │ COAGULATION END TIME BASED ON         │
      │ MEASUREMENT RESULT OF WAVELENGTH λ1   │
      └──────────────────────────────────────┘
                      │
                      ▼                        S102
   NO        ╱   COAGULATION END TIME  ╲
  ┌──────────╲      CAN BE SET ?       ╱
  │           ╲                       ╱
  │                   │ YES                    S103
  │          ┌──────────────────────────────┐
  │          │ CALCULATE COAGULATION TIME    │
  │          │ BASED ON COAGULATION END TIME │
  │          └──────────────────────────────┘
  │                   │                        S104
  │          ┌──────────────────────────────┐
  │          │ CALCULATE ABSORBANCE OF       │
  │          │ WAVELENGTH λ1 AND λ2          │
  │          └──────────────────────────────┘
  │                   │
  │                   ▼                        S105
  │           ╱   COAGULATION      ╲
  │           ╲ REACTION OCCURRED ? ╱───────┐ YES
  │            ╲                   ╱          │
  │                  │ NO                     │
  └──────────────────┤                        │
                     ▼   S106                  │
            ┌─────────────────┐                │
            │   GIVE FLAG      │               │
            └─────────────────┘                │
                     │◄────────────────────────┘
                   RETURN
```

EP 2 775 292 B1

EP 2 775 292 B1

# FIG. 10

EP 2 775 292 B1

# F I G. 1 1

# F I G. 1 2

<MEASUREMENT UNIT>

START

PREPARE MEASUREMENT SPECIMEN — S11

MEASURE MEASUREMENT SPECIMEN (MEASUREMENT TIME PERIOD T1) — S12

TRANSMIT MEASUREMENT RESULT — S13

RE-MEASUREMENT NECESSARY ? — S14
— NO
— YES

MEASURE MEASUREMENT SPECIMEN (MEASUREMENT TIME PERIOD T2) — S15

TRANSMIT MEASUREMENT RESULT — S16

END

<CONTROL DEVICE>

START

MEASUREMENT RESULT RECEIVED ? — S21
— NO
— YES

ANALYSIS PROCESS — S22

DISPLAY PROCESS — S23

RE-MEASUREMENT ALREADY PERFORMED ? — S31
— YES
— NO

RE-MEASUREMENT INSTRUCTION RECEIVED ? — S32
— YES
— NO

TRANSMIT RE-MEASUREMENT ORDER — S33

TRANSMIT INFORMATION INDICATING UNNECESSITY OF RE-MEASUREMENT — S34

END

FIG. 13A

ABSORBANCE

λ2(575nm)

λ1(660nm)

A21
A11

REAGENT
ADDITION
(FIRST TIME)

COAGULATION
START TIME
(FIRST TIME)

COAGULATION
END TIME
(FIRST TIME)

MEASUREMENT
END TIME
(FIRST TIME)

ELAPSED TIME

FIG. 13B

ABSORBANCE

A21

A11

λ2(575nm)

λ1(660nm)

A20
A10

REAGENT
ADDITION
(SECOND TIME)

COAGULATION
START TIME
(SECOND TIME)

COAGULATION
END TIME
(SECOND TIME)

MEASUREMENT
END TIME
(SECOND TIME)

ELAPSED TIME

EP 2 775 292 B1

F I G. 1 4

FIG. 15B

ANALYSIS PROCESS

START

S101 SET COAGULATION START TIME AND COAGULATION END TIME BASED ON MEASUREMENT RESULT OF WAVELENGTH λ1

S102 COAGULATION END TIME CAN BE SET ?
NO → (to GIVE FLAG)
YES

S104 CALCULATE ABSORBANCE OF WAVELENGTH λ1 AND λ2

S105 COAGULATION REACTION OCCURRED ?
NO → TO S21
YES

S106 GIVE FLAG

S107 CALCULATE COAGULATION TIME AND TRANSMIT STOP INSTRUCTION

RETURN

FIG. 15A

<CONTROL DEVICE>

START

S21 MEASUREMENT RESULT RECEIVED ?
NO
YES

S22 ANALYSIS PROCESS

S23 DISPLAY PROCESS

END

<MEASUREMENT UNIT>

START

S11 PREPARE MEASUREMENT SPECIMEN

S12 MEASURE MEASUREMENT SPECIMEN (MEASUREMENT TIME PERIOD T3)

S17 TRANSMIT MEASUREMENT RESULT

S18 STOP INSTRUCTION RECEIVED ?
NO
YES

S19 STOP MEASUREMENT

END

# F I G. 1 6 A

```
        START
                              S101
  SET COAGULATION START TIME AND
    COAGULATION END TIME BASED ON
MEASUREMENT RESULT OF WAVELENGTH λ1

                              S102
        COAGULATION END TIME
NO         CAN BE SET ?
             ↓ YES         S103
  CALCULATE COAGULATION TIME BASED ON
      COAGULATION END TIME
                              S104
  CALCULATE ABSORBANCE OF WAVELENGTH
      λ2 AND λ3

                              S105
        COAGULATION
    REACTION OCCURRED ?       YES
             NO
                              S106
        GIVE FLAG

        RETURN
```

# F I G. 1 6 B

```
        START
                              S101
  SET COAGULATION START TIME AND
   COAGULATION END TIME BASED ON
MEASUREMENT RESULT OF WAVELENGTH λ1

                              S102
       COAGULATION END TIME
NO        CAN BE SET ?
             ↓ YES
                              S104
  CALCULATE ABSORBANCE OF WAVELENGTH
      λ1 AND λ2

                              S105
       COAGULATION
   REACTION OCCURRED ?        YES
                                         S103
             NO              CALCULATE COAGULATION
                                 TIME BASED ON
                             COAGULATION END TIME
                              S106
        GIVE FLAG

        RETURN
```

FIG. 17A    FIG. 17B

**REFERENCES CITED IN THE DESCRIPTION**

**Patent documents cited in the description**

- US 2007222973 A

- JP 2010217059 A